# EUROPEAN PATENT SPECIFICATION

(11) **EP 2 419 132 B1**
(45) Date of publication and mention of the grant of the patent: **25.06.2014**
(21) Application number: 10713677.2
(22) Date of filing: 15.04.2010
(51) Int. Cl.: A61K 39/255

(54) **RECOMBINANT AVIAN HERPES VIRUS VECTORS AND VACCINE FOR IMMUNIZING WATERFOWL SPECIES**
REKOMBINANTES HÜHNE-HERPESVIRUS UND IMPFSTOFF ZUR IMMUNISIERUNG VON WASSERVÖGELN
VIRUS HERPÈS AVIAIRE RECOMBINANT ET VACCIN POUR L'IMMUNISATION DES OISEAUX AQUATIQUES

(30) Priority: 15.04.2009 US 169459 P; 18.06.2009 US 218280 P; 20.07.2009 US 226970 P
(43) Date of publication of application: 22.02.2012
(73) Proprietor: Ceva Sante Animale, 33501 Libourne Cedex (FR)
(72) Inventor: GARDIN, Yannick, F-49460 Cantenay Epinard (FR); PALYA, Vilmos, H-1223 Budapest (HU)
(74) Representative: Becker, Philippe
(86) International application number: PCT/EP2010/054991
(87) International publication number: WO 2010/119112

(56) References cited:
- EP-A1- 1 026 246
- WO-A1-01/05988
- WO-A1-97/20576
- WO-A2-03/064595
- WO-A2-2008/121329
- RAUW FABIENNE ET AL: "Improved vaccination against Newcastle disease by an in ovo recombinant HVT-ND combined with an adjuvanted live vaccine at day-old" VACCINE, vol. 28, no. 3, January 2010 (2010-01), pages 823-833, XP026793755 ISSN: 0264-410X

## Description

### Technical field of the invention

The present invention relates generally to the fields of immunology and vaccine technology. The present invention specifically relates to vaccines for waterfowl immunization based on live recombinant Marek's disease virus (hereinafter referred to as rMDV), and more specifically recombinant herpes virus of turkeys (hereinafter referred to as rHVT virus), which incorporates at least one nucleotide sequence encoding and expressing an antigenic peptide of a pathogenic agent of waterfowl. Methods of immunization are also provided.

### Background of the invention

Marek's disease virus (MDV) is the causative agent of Marek's disease, a common lymphoproliferative disease of chickens. Marek's disease (MD) is a highly contagious disease in chicken flocks worldwide. Marek's disease virus (MDV) has been identified as the etiologic agent of MD. MDV infects bursa-derived and thymus-derived lymphocytes in chickens, and may subsequently induce a lymphoma of thymus-derived lymphocytes. MDV is a designation of a family of avian herpes viruses. For example, MDV (MDV1) is a virulent strain of herpes virus in chickens, SB-1 (MDV2) is a naturally attenuated herpes virus strain in chickens, and HVT (MDV3) is a non pathogenic herpes virus commonly found in turkeys.

In terms of host range, only chickens and turkeys were reported as being naturally infected by MDV and HVT. Other birds were however up to now found refractory to the disease or infection. Vaccination of chicken using recombinant MDV has been disclosed in WO2008/121329 ; WO01/05988 ; WO03/064595 or EP1026246.

Many waterfowl birds like ducks and geese are of economic importance, as they are raised for meat, eggs, and feathers. Hence immunization of these birds against common pathogens is a requirement to improve the quality of this poultry. Further, wild waterfowl, the natural hosts of all known influenza A viruses, are the sources of viruses that cause sporadic outbreaks of highly fatal disease in domestic poultry. The recent emergence of highly pathogenic avian influenza (HPAI) strains in poultry and their subsequent transmission to humans in Southeast Asia, with frequent outbreaks in poultry leading to the destruction of hundreds of millions of animals, has raised concerns about the potential pandemic spread of lethal disease. In order to contain such transmission of pathogenic agents, it is important to immunize the natural hosts, namely the waterfowl.

The Applicant has surprisingly found that Marek's disease virus (MDV) is capable of infecting species of waterfowl and can replicate, maintain and survive in the infected waterfowl hosts in the state of latent infection or persistent infection, thereby providing effective vaccination means.

The vaccines according to the present invention are thus able to achieve effective vaccination of waterfowl, and have superior vaccination properties in that they can raise long duration immunity to immunized waterfowl hosts. Therefore, adequate vaccination effect is advantageously conferred to the waterfowl hosts by administering recombinant HVT or MDV into which genes of foreign antigens have been inserted.

### Summary of the invention

The present invention provides a recombinant Marek's disease virus (rMDV), specifically a recombinant herpes virus of turkey (rHVT) for immunizing waterfowl species, comprising one or more heterologous or homologous nucleotide sequences incorporated into the viral genome, capable of encoding and expressing at least one antigenic peptide of a pathogenic agent in cells of waterfowl species, and further capable of inducing a stable and long lasting immune response against pathogens causing infection in waterfowl.

The present invention also relates to vaccines for immunizing waterfowl species comprising an effective immunizing amount of the rHVT or rMDV optionally formulated with solvents, stabilizers, adjuvants, diluents, preservatives and other excipients.

The present invention further relates to methods of immunizing waterfowl species and vaccination kits.

### Brief summary of the Figures

**Figure 1** results of HVT specific PCR (samples collected at 4 weeks post-vaccination). Abbreviations used: CA-ducks vaccinated with cell-associated HVT vaccine; FD-ducks vaccinated with freeze-dried HVT vaccine. Samples are identified as vaccine abbreviation/ organ sampled.
**Figures 2A-2D** correspond to the nucleotide sequences and deduced amino acid sequences of the homology plasmid, p45CMVH5HUNMod4999 (SEQ ID NO: 1).
**Figure 3A** shows a schematic diagram of the turkey herpes virus (HVT) genome and the location of the Unique Long (UL) 44, UL 45 and UL 46 genes are marked. The Donor gene was inserted at a PCR generated *Sfi*I site between UL 45 and UL 46 in the HVT genome.
**Figure 3B** shows a portion of UL 44, the entire UL 45 and a portion of UL 46 that can be isolated on a 1.9-kilobase (kb) *Eco*RV-*Xho*I fragment of the HVT genome. A box shows where a probe (insertion site probe or 45/46 probe) designed to bind to sequences flanking the insertion site anneals to a 1.0-kb *Pvu*I-*Pvu*I fragment in a Southern blot.
**Figure 3C** shows the strategy of isolation of the insertion site sequence.
**Figure 4A** shows the isolation of the *Hemagglutinin (HA)* gene in the avian influenza virus H5 subtype genome which consists of eight segments of negative-sense, single stranded RNA. Also shown are the proteins expressed from each segment and the functions of these proteins.
**Figure 4B** shows the *HA* gene that was amplified from the avian influenza virus RNA genome by reverse transcriptase-polymerase chain reaction using a forward primer and a reverse primer, which adds *Bam*HI and *Sal*I restriction enzyme sites to 5' and 3' ends, respectively. The cleavage site of the *HA* gene was altered to typical cleavage site sequence of low pathogenic avian influenza strains. Modification of the cleavage site was conducted by polymerase chain reaction
**Figure 5** shows the construction of intermediate plasmid pGICMVpA. The cytomegalovirus immediate early promoter (CMV promoter) and the SV40 polyA signal sequence were inserted into the pUC18 vector.
**Figure 6** shows the construction of the homology plasmid p45CMVH5HUNMod4999.
**Figure 7** shows the preparation of the regulated avian Influenza-Marek's Disease Vaccine, H5 Subtype, Serotype 3, Live Marek's Disease Vector.
**Figure 8** shows the production of the regulated avian Influenza-Marek's Disease Vaccine, H5 Subtype, Serotype 3, Live Marek's Disease Vector.
**Figure 9** shows comparative results obtained from efficacy testing of recombinant rHVT-Al/H5 vaccine in Muscovy ducks when challenged with H5N1 highly pathogenic Avian Influenza clade 2 A/Duck/Hungary/1180/2006 vis-a-vis unvaccinated control birds.

### Definitions

In the present invention, the term "recombinant" refers to recombinant DNA technology, also called gene cloning or molecular cloning, which refers to techniques of transfer of DNA from one organism to another.

The terms "recombinant herpes virus of turkeys" (rHVT) and/or recombinant Marek Disease virus" (rMDV) are referred herein to an existing herpesvirus of turkeys whose genome has been modified by insertion of at least one heterologous nucleic acid sequence, *i.e*., DNA which corresponds to a gene or part thereof not identical to the nucleic acid sequence of a gene naturally present in HVT or MDV. Alternatively, the rHVT or rMDV may be genetically modified by the incorporation into the virus genome of a homologous nucleic acid sequence, *i.e.*, DNA which corresponds to a gene or part thereof identical of a gene naturally present in HVT or MDV. It will be understood that the resulting recombinant HVT (rHVT) and/or recombinant MDV (rMDV) can be manufactured by a variety of methods, and once made, can be reproduced without use of further recombinant DNA technology. The structure of the "recombinant herpes virus of turkeys" is therefore described in terms of DNA insertion.

The term "waterfowl" species according to the present invention are intended to mean birds that are adapted for swimming, floating on the water surface, and in some cases diving in shallow water. More precisely, waterfowl species comprise birds of the order of the Anseriformes, of the Anatidae family, such as ducks, geese and swans, and belong to Anatinae, Anserinae, Stictonettinae, Tadorninae, or Dendrocygninae subfamily. For example, the order Anseriformes contains about 170 species of birds in three families: the Anhimidae (the screamers), Anseranatidae (the Magpie-goose), and the Anatidae, which includes over 160 species of waterfowl. All species in the order are highly adapted for an aquatic existence at the water surface. All are web-footed for efficient swimming (although some have subsequently become mainly terrestrial). The scope of the invention is extended to all birds covered under these broad families.

The term "adjuvant" is defined as one or more substances that cause stimulation of the immune system. Adjuvants are agents that non specifically increase an immune response to a particular antigen, thus reducing the quantity of antigen necessary in any given vaccine, and/or the frequency of administration necessary in order to generate an adequate immune response to the antigen of interest. In this context, an adjuvant is used to enhance an immune response to the vaccines of the present invention. An adjuvant may be administered to the target animal before, in combination with, or after the administration of the vaccine.

As used herein, the word "pathogenic" means the causing by a biological agent of a disease or illness to its host, in this case specifically a waterfowl host.

The terms "untranslated regions" as used herein refer to a region of nucleotides that has no ORF and do not define an amino acid sequence of protein to be expressed by translation, and a region of nucleotides in which the ORF is not involved in any of transcription, translation, or protein expression. The nucleotide sequences contained in this region may be those that have substitutions, deletions, or additions of bases, unless they include an ORF.

### Detailed description of the invention

The present invention thus provides a recombinant Marek's disease virus (rMDV), specifically a recombinant herpes virus of turkey (rHVT) for immunizing waterfowl species, comprising one or more heterologous or homologous nucleotide sequences incorporated into the viral genome, capable of encoding and expressing at least one antigenic peptide of a pathogenic agent in cells of waterfowl species, and further capable of inducing a stable and long lasting immune response against pathogens causing infection in waterfowl.

As long as being non-pathogenic to waterfowl, any Marek's disease virus (MDV) (for example serotypes 1, 2, 3, CVI988, SB1 etc.) or specifically herpes virus of turkeys (HVT) can be used in the present invention. MDV and HVT for use in the present invention may thus include, but are not limited to, naturally occurring ones or those available from depositories like ATCC, CNCM, etc... For instance, FC126 (ATCC VR-584B), PB-THV1, H-2, YT-7, WTHV-1, or EPRS-26 strain is suitable for the backbone virus. Among these, FC126 is most preferably used in the present invention because of its safe use in avian.

The genes of interest targeted for insertion into the genome of HVT or MDV may be homologous or heterologous nucleotide sequences which are obtained from any pathogenic organism which are capable of causing infection in waterfowl species. Typically, the genes of interest may be derived from pathogens which cause diseases that have an economic impact on the poultry industry. The genes of interest may also be derived from pathogens which are maintained in waterfowl and are transmitted to other poultry and non poultry species through the waterfowl hosts. The genes may further be derived from organisms for which there are existing vaccines, and because of the novel advantages of the vectoring technology, the HVT or MDV derived vaccines are superior. In addition, the genes of interest may be derived from pathogens for which there is currently no vaccine but where there is a requirement for controlling the disease. Typically, the genes of interest encode immunogenic peptides of a pathogen, and preferably represent surface proteins, secreted proteins and structural proteins.

The homologous or heterologous nucleotide sequence for insertion into the MDV or HVT genome may thus be any sequence coding for an antigenic peptide of waterfowl pathogenic agents. The nucleic acid sequence according to the present invention can be derived from any source, *e.g*., viral, prokaryotic, eukaryotic or synthetic. Said nucleic acid sequence may be derived from a pathogen, preferably a pathogen of waterfowl species, which after insertion into the viral genome can be stably expressed to induce immunity against waterfowl diseases.

The heterologous or homologous nucleotide sequence may encode for example an antigenic peptide derived from avian influenza virus, avian paramyxovirus type 1 (Newcastle disease virus, NDV), avian metapneumovirus, Marek's disease virus, Gumboro disease virus (infectious bursal disease virus: IBDV), goose and Muscovy duck parvoviruses, duck virus enteritis herpes virus, goose herpes virus, duck hepatitis virus type 1, 2 and 3, goose haemorrhagic polyomavirus, duck and goose adenoviruses, goose and duck circoviruses, West Nile virus, goose and Muscovy duck reoviruses, Escherichia coli, Salmonella species, Pasteurella multocida, Riemerella anatipestifer, Ornithobacterium rhinotracheale, Mycoplasma gallisepticum, Mycoplasma synoviae, Mycoplasmas microorganisms infecting waterfowl species or coccidian.

In a first embodiment, such heterologous or homologous nucleotide sequence may be for example the surface protein hemagglutinin (HA) and/or neuraminidase (NA) of the influenza virus, the protein of the Marek's disease virus, especially gB, gC, gD, gH and/or gL, the F and/or HN protein of the Newcastle disease virus, the VP1, VP2, and/or VP3 protein of goose and Muscovy duck parvoviruses, any of the structural proteins of goose and Muscovy duck reoviruses, the VP1 protein of goose haemorrhagic polyomavirus, the antigens of goose and duck virus enteritis herpes virus, the antigens of duck and goose adenovirus, the capsid proteins of circoviruses, or the VP2 protein of IBDV.

In another specific embodiment, such heterologous or homologous nucleotide may encode a non-glycosylated NXB amino acid sequence wherein N is an asparagine, X is any amino acid other than proline, and B is a threonine as described in US 6,936,707, and which is capable of exhibiting a high immunogenicity.

In another embodiment, the heterologous or homologous nucleotide sequence may also be fusion proteins comprising a peptide having the antigenicity of Mycoplasma gallisepticum and a peptide derived from herpes virus outer membrane protein as described in US 7,348,422.

In yet another preferred embodiment the rHVT or rMDV may further comprise a second heterologous nucleotide sequences encoding for a second antigen or immune modulators such as lymphokines, interferons, or avian cytokines.

In order to construct a recombinant MDV or HVT virus of the present invention, initially, the above virus is propagated in a suitable host cell and then the genomic DNA is obtained. The host and the conditions for propagating the virus are selected as appropriate. As host cells, cells derived from chicken are preferred, and CEF (chick embryo fibroblast), chicken kidney cells, and the like, can be used. It may be cultured in a culture medium such as Eagle's MEM, Leibowitz-L-15/McCoy 5A (1:1 mixture) culture medium at about 37°C for 3 to 4 days.

DNA is extracted from the virus infected cells cultured as above according to a conventional method. Thus, the cells grown in monolayers are scraped, and then spun to harvest the supernatant. After protein is denatured in the lysis buffer and removed, DNA is extracted with phenol and ethanol.

Gene cloning and plasmid construction is well known to one person of ordinary skill in the art and may be essentially performed by the standard molecular biology techniques (Molecular Cloning: A Laboratory Manual. 3rd Edition, Cold Spring Harbor Laboratory Press, Woodbury, N.Y. 2001). Sequences for insertion into the virus may be derived from any suitable plasmid, cosmid or phage, plasmids being most preferred, and containing at least one heterologous or homologous nucleic acid sequence, if desired operably linked to a promoter.

The promoter used may be either a synthetic or natural, endogenous or heterologous promoter, and is not limited as long as it can effectively function in cells of waterfowls infected with rHVT or rMDV. Hence the choice of a promoter extends to any eukaryotic, prokaryotic or viral promoter capable of directing gene transcription in cells of waterfowl infected by the recombinant HVT or MDV. Various promoters may be used, such as for example the chicken beta-actin promoter, the modified chicken beta-actin promoter as described in US 6,866,852, the immediate early CMV (cytomegalovirus) promoter, the SV40 early promoter, the pseudorabies virus immediate early promoter (PRV IE), the thymidine kinase (TK) or glycoprotein X (gX) promoter, the herpes simplex alpha-4 promoter, the protein gB promoter of the virus of Marek's disease, the promoter originating from the RSV virus (Rous sarcoma virus) LTR sequence, as well as MDV or HVT promoters, such as the promoters of the gB, gC, TK, RR2, or any fragment thereof which retain a promoter activity. Among the promoters mentioned, preferred promoters are strong promoters such as for example, the chicken beta-actin promoter, the immediate early CMV (cytomegalovirus) promoter, and the SV40 early promoter, which have shown some degree of efficacy, as well as promoters belonging to some genes of the Marek viruses, in particular of serotype 3.

The homologous or heterologous nucleotide sequences coding for the antigens of interest for the said waterfowl disease, may thus be operably linked to a promoter as described above and further inserted into the insertion region of the viral genome. Insertion region is understood to mean, in particular, insertion without deletion or with deletion of a few bases for the intergenic regions, and with total or partial deletion or without deletion for the ORFs.

The homologous or heterologous nucleotide and promoter sequence may be inserted for example into the TK region as described by Ross L. Et al. Gen. Virol., 74:371-377 (1993). Preferably, the nucleotide and promoter sequences are inserted into an untranslated region of the viral genome. Generally, an untranslated region of the genomic DNA is identified and then the homologous or heterologous nucleotide sequence described above is inserted into the region.

Examples of insertion sites are selected between UL44 and UL45, between UL45 and UL46, between UL41 and UL42, between UL40 and UL41, a region located downstream of the gB gene, between UL53 and UL54, or between UL36 and UL37. They are described for HSV-1 in the Proceedings of the 16th Herpes Virus Workshop (held at Pacific Grove in California, U.S.A., on Jul. 7-12, 1991). Among these, preferred sites are between UL45 and UL46 and in a region located downstream of the gB gene for which homology, and more preferably between UL45 and UL46.

Said nucleic acid sequence and promoter are introduced into a fragment of genomic HVT or MDV DNA containing insertion region sequences as defined herein subcloned in the recombinant DNA molecule. The insertion region sequences which flank the heterologous or homologous nucleic acid sequence are generally of appropriate length so as to allow in vivo homologous recombination with the viral genome. If desired, a construct can be made which contains two or more different heterologous or homologous nucleic acid sequences, *e.g*., derived from the same or different pathogens, said sequences being flanked by insertion region sequences of HVT or MDV defined herein. Such a recombinant DNA molecule can be employed to produce recombinant HVT or MDV which expresses two or more different antigenic peptides to provide a multivalent vaccine. Secondly, cells may be transfected with viral DNA in the presence of the recombinant DNA molecule containing the heterologous or homologous nucleic acid sequence flanked by appropriate HVT sequences whereby recombination occurs between the insertion region sequences in the recombinant DNA molecule and the insertion region sequences in HVT. Recombination can also be brought about by transfecting the infected cells with a nucleic acid sequence containing the heterologous or homologous nucleic acid sequence flanked by appropriate flanking insertion region sequences without recombinant DNA molecule sequences.

In order to insert a foreign gene in an untranslated region of HVT or MDV, it is necessary to clone a sequence containing the untranslated region into a plasmid in advance, but the plasmid is not limited. For example, there can be mentioned plasmids such as pBR322, pBR325, pBR327, pBR328, pUC18, pUC19, pUC7, pUC8, and pUC9, and phages such as lambda phage and M13 phage, and cosmids such as pHC79.

An untranslated region obtained as mentioned above is integrated into these plasmids according to a conventional method. In order to insert one or more heterologous or homologous nucleotide sequence into the untranslated region integrated as above, mutation may be carried out at a specific site of the untranslated region that was cloned in a plasmid as above to make a new cleavage site for restriction enzymes, and then the foreign gene is inserted into the site. A method of carrying out mutation may be a conventional method, and a method commonly used by a person skilled in the art such as in vitro mutagenesis and PCR can be used. Thus, in the PCR method, a mutation such as the deletion, replacement, or addition of 1 to 2 nucleotides in the PCR primer is carried out, and the primer is then used to create a mutation.

A plasmid in which one or more heterologous or homologous nucleotide sequence has been inserted into the HVT or MDV untranslated region obtained as above is introduced into an HVT-infected cell using electroporation, calcium phosphate, a lipofectin-based method, a gene gun method or the like. Because of the high efficiency of introduction, electroporation or a method employing lipofectin is preferred. When the amount of the plasmid to be introduced is in the range of 0.1 to 1000 µg, the efficiency of generation of recombinant viruses by recombination between the homologous regions of HVT-DNA and the plasmid becomes high in cells.

Recombinant viral progeny is thereafter produced in cell culture and can be selected genotypically or phenotypically using known techniques of selection, *e.g*., by hybridization, detecting enzyme activity encoded by a gene co-integrated along with the heterologous or homologous nucleic acid sequence or detecting the antigenic peptide expressed by the recombinant HVT or MDV immunologically. The selected recombinant HVT or MDV can be cultured on a large scale in cell culture after which, recombinant HVT or MDV containing peptides can be collected.

For enabling selection of rHVT or rMDV, several markers may be integrated into the untranslated region of the plasmid. Examples of such an enzyme gene include β-galactosidase gene. Therefore, by culturing cells infected with a virus that has integrated such a gene, in a culture medium supplemented with a specific substrate, it is possible to select virus-infected cells. By repeating this procedure, recombinant viruses can be purified.

The resulting recombinant viruses of the present invention may be propagated in cell cultures in which said recombinant virus can propagate and grow. After required growth of the viruses is achieved the cells are detached from the wells using a scraper or with trypsin and separate the infected cells are separated from the supernatant by centrifugation.

In preferred embodiments of the invention, CEF, embryonated egg, chicken kidney cell, and the like may be used as the host cells for the propagation of rHVT or rMDV. Recombinant viruses of the present invention may be cultured in a culture medium such as Eagle's MEM, Leibowitz-L-15/McCoy 5A (1:1 mixture) culture medium at about 37° C for 3 to 4 days. The infected cells thus obtained are suspended in a culture medium containing 10% dimethyl sulfoxide (DMSO) and stored frozen under liquid nitrogen.

For use as vaccine, the above frozen product may be dissolved in 100 volumes of phosphate buffer prior to use. Stabilizers and other components for use in storing said infected cells under liquid nitrogen are not limited as long as they enable the virus-infected cells to grow stably and they are pharmaceutically acceptable.

The present invention also provides a vaccine for immunizing waterfowl species comprising an effective amount of recombinant MDV or HVT carrying in their genome at least one heterologous or homologous nucleotide sequence encoding and expressing in cells of waterfowl species at least one antigenic protein as described above.

A recombinant HVT or MDV according to the invention may be preferably used as a live vaccine although other alternatives like inactivated vaccines or attenuated vaccines are well within the skill of a person skilled in the art.

As demonstrated by the Applicant, rHVT or rMDV vector elicited long lasting protective immunity against waterfowl diseases. Particularly, as demonstrated in the Examples, the recombinant viral vector is capable of infecting and replicating in waterfowl cells in bursa, spleen, thymus and feather follicles. In particular, rHVT or rMDV have the property of permanently surviving in the body of the vaccinated waterfowl in the state of latent infection and thereby generating a long-lasting protection during the whole life of vaccinated waterfowl.

Recombinant HVT or MDV according to the invention may contain and express one or more different heterologous peptides or proteins can serve as a monovalent or multivalent vaccine. They may also be mixed with other viral or bacteria so as to constitute polyvalent or multivalent immunogenic preparations or multivalent vaccines. The viral or bacterial are selected from avian pathogens characteristic of the waterfowl species to be vaccinated. The present invention still further also relates to a polyvalent or multivalent vaccine comprising, as a mixture or to be mixed, at least two live rHVT vaccines as defined above, which vaccines comprise different inserted sequences, in particular from different pathogens. For example the vaccine of the invention may incorporate more than one antigen derived from more than one waterfowl pathogens, such as an antigen derived from the avian influenza (Al) virus and from the Newcastle disease virus. Alternatively, the multivalent vaccine may comprise a rHVT or rMDV vector carrying more than one heterologous or homologous nucleotide sequence encoding an antigenic peptide or protein for example under the control of a bidirectional promoter.

The vaccine according to the present invention may further comprise a suitable solvent, such as for example an aqueous buffer or a phosphate buffer. Preferably, the vaccine also comprises an adjuvant. Adjuvants of the present invention may be obtained from any of a number of sources including from natural sources, recombinant sources, and/or be chemically synthesized, etc. Examples of adjuvants include chemical and polypeptide immunostimulants which enhance the immune system response to antigens. Such adjuvants may include for example, Freund's complete or incomplete adjuvant, mineral gels, aluminum compounds such as aluminum hydroxide, aluminum phosphate, and alum; surfactants, such as hexadecylamine, octadecylamine, lysolecithin, dimethyldioctadecylammonium bromide, N,N-dioctadecyl-N',N'-bis (2-hydroxymethyl) propanediamine, methoxyhexadecylglycerol, and pluronic polyols; polyanions, such as pyran, dextran sulfate, poly IC, polyacrylic acid; peptides, such as muramyl dipeptide, dimethylglycine and tuftsin; and oil emulsions, plant and animal oils as well as mineral oils, water in oil emulsions bacterial toxins, chitin derivatives and chitosan, polymers, ISCOM's (immune stimulating complexes), vitamins and minerals (including but not limited to: vitamin E, vitamin A, selenium, and vitamin B12), Quil A (saponins), polymers of acrylic acid crosslinked with polyalkenyl ethers or divinyl glycol, bacterial and fungal cell wall components (e.g., lipopolysaccarides, lipoproteins, glycoproteins, muramylpeptides; beta-1,3/1,6-glucans), various complex carbohydrates derived from plants (e.g., glycans, acemannan), various proteins and peptides derived from animals (e.g., hormones, cytokines, co-stimulatory factors), and novel nucleic acids derived from viruses and other sources (e.g., double stranded RNA, CpG), and the like which are administered with the vaccine in an amount sufficient to enhance the immune response. A preferred adjuvant according to the present invention is the chitosan. In addition, any number of combinations of the aforementioned substances may provide an adjuvant effect, and therefore, can form an adjuvant of the present invention.

The vaccines of the present invention may further be formulated with one or more further additives to maintain isotonicity, physiological pH and stability, for example, a buffer such as physiological saline (0.85%), phosphate-buffered saline (PBS), citrate buffers, Tris(hydroxymethyl aminomethane (TRIS), Tris-buffered saline and the like, and/or a preservative such as thiomersol, formalin, glutaraldehyde, or an antibiotic, for example, neomycin or streptomycin, etc.

The method of administration may be selected by the skilled artisan. Waterfowl vaccines of the present invention is readily administered by any route including oral, ocular(e.g., by eyedrop), oculo-nasal administration using aerosol, intranasal, Cloacal in feed, in water, or by spray, in ovo, topically, or by injection (e.g., intravenous, subcutaneous, intramuscular, intraorbital, intraocular, intradermal, and/or intraperitoneal) vaccination. The skilled artisan will appreciate that the vaccine composition is preferably formulated appropriately for each type of route of administration. For instance, the vaccine composition may be administered to post-hatch, young (few days to several weeks old) birds via drinking water, spraying or eye drops. In ovo administration is also contemplated herein. For example, embryos may be inoculated at a suitable time before hatch.

Each vaccine dose may contain a suitable dose sufficient to elicit a protective immune response in waterfowl species. Optimization of such dose is well known in the art. The amount of antigen per dose may be determined by known methods using antigen/antibody reactions, for example by the ELISA method.

The program for immunizing or vaccinating waterfowl may in particular comprise one or two administration(s) to waterfowl. A booster administration may also be given. Those skilled in the art have the competence necessary to define precisely the number of injections and the doses of each vaccine to be used for each vaccination protocol.

The vaccines of the present invention are further advantageous in that they can be used to ensure total protection of waterfowl against various diseases by immunizing birds as young as one-day old, at very low doses, without any side effects.

More particularly, the vaccine confers to waterfowl species at least 20% to 30% protection against the challenge of infectious diseases, after 2 weeks of vaccination, and at least 50% to 60% protection against the challenge of infectious diseases, after 5 to 6 weeks of vaccination. Preferably, the vaccine according to the present invention confers to waterfowl species up to 80% to 100% protection against the challenge of infectious diseases, after 5 to 6 weeks of vaccination.

The above described active immunization protocol against specific pathogens may be applicable to elicit protective immunity in waterfowl species. Further, waterfowl already infected with a specific pathogen can be also be treated with antiserum comprising antibodies evoked by a recombinant HVT or MDV carrying a heterologous or homologous gene encoding pathogenic antigen, according to the present invention. Antiserum directed against a recombinant HVT or MDV according to the invention can be prepared by immunizing waterfowl with an effective amount of said recombinant HVT in order to elicit an appropriate immune response. Thereafter the animals are bled and antiserum can be prepared. The present invention thus comprises antiserum directed against rHVT or rMDV obtainable by immunizing waterfowl with an effective amount of rHVT or rMDV and by bleeding the waterfowl for use as a veterinary drug.

In a preferred embodiment of the present invention, rHVT or rMDV carry and express a nucleotide sequence encoding the hemagglutinin (HA) glycoprotein of avian influenza virus under the control of an endogenous or heterologous promoter. Such rHVT or rMDV containing a heterologous nucleotide sequence encoding HA is thus particularly useful for use as a veterinary drug for protecting waterfowl against influenza.

Influenza viruses that infect birds are called avian influenza viruses, or influenza A. Only influenza A viruses infect birds, and all known subtypes of influenza A viruses can infect birds. Avian influenza viruses circulate among birds worldwide. Certain birds, particularly waterfowls, act as hosts for influenza viruses by carrying the virus in their intestines and shedding it. Infected birds shed virus in saliva, nasal secretions, and feces. Susceptible birds can become infected with avian influenza virus when they have contact with contaminated nasal, respiratory, or fecal material from infected birds. Fecal-to-oral transmission is the most common mode of spread between birds. Most often, the wild birds that are host to the virus do not get sick, but they can spread influenza to other birds. Infection with certain avian influenza A viruses (for example, some H5 and H7 strains) can cause widespread disease and death among some species of domesticated birds. Domesticated birds may become infected with avian influenza virus through direct contact with infected waterfowl or other infected poultry, or through contact with surfaces (such as dirt or cages) or materials (such as water or feed) that have been contaminated with virus. People, vehicles, and other inanimate objects such as cages can be vectors for the spread of influenza virus from one farm to another. When this happens, avian influenza outbreaks can occur among poultry. Influenza A viruses can also infect humans as well as other hosts including pigs, whales, horses, seals and so on.

Avian influenza viruses are classified in the family Orthomyxoviridae, genus Influenza virus A. The genome of the avian influenza virus consists of eight segments of single-stranded, negative-sense RNA. The viral genome encodes ten proteins, of which eight proteins are structural proteins including HA and neuraminidase (NA), and two proteins are non structural. Influenza A viruses are divided into subtypes based on antigenicity of HA and NA proteins. There are 16 HA antigens and nine NA antigens recognized. HA is considered the major antigen that can elicit protective antibodies in birds.

Avian influenza A viruses are categorized into two pathotypes based on their pathogenicity: highly pathogenic avian influenza (HPAI) viruses and low pathogenic avian influenza (LPAI) viruses. Most avian influenza viruses are of low virulence, but a few viruses of H5 and H7 subtypes can cause severe systemic disease that results in high mortality.

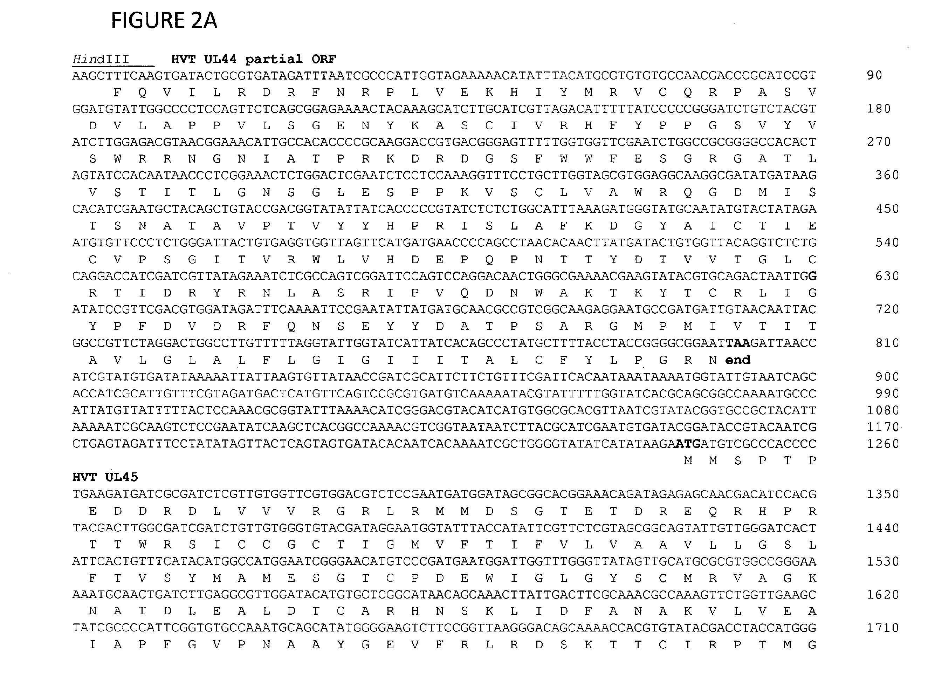

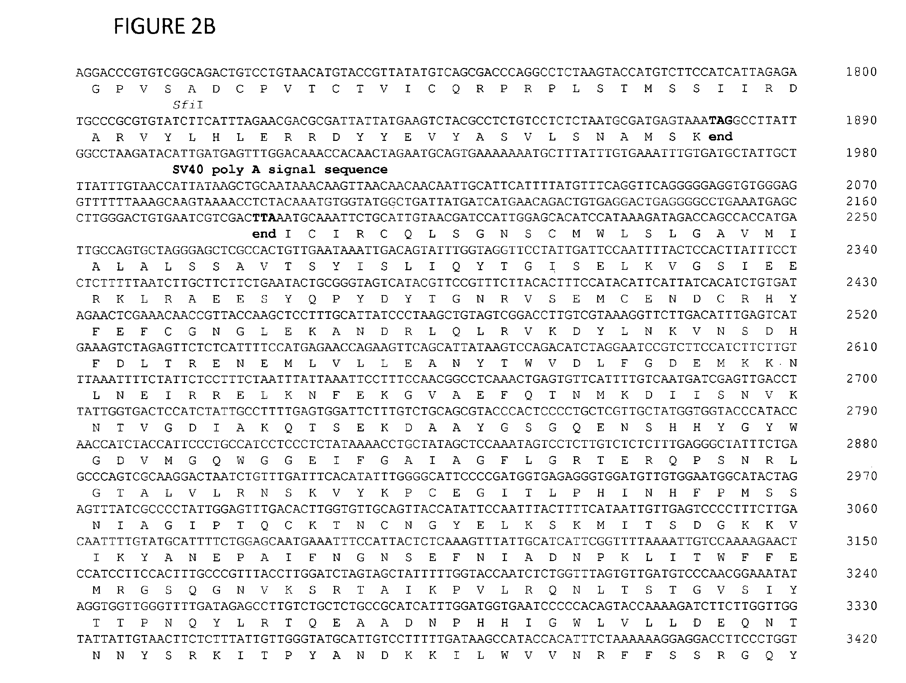

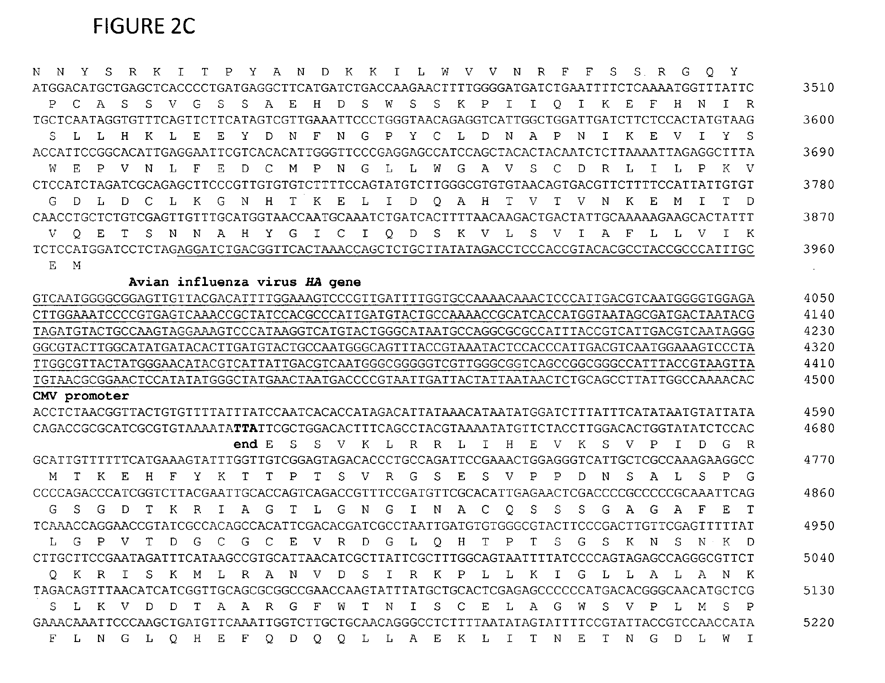

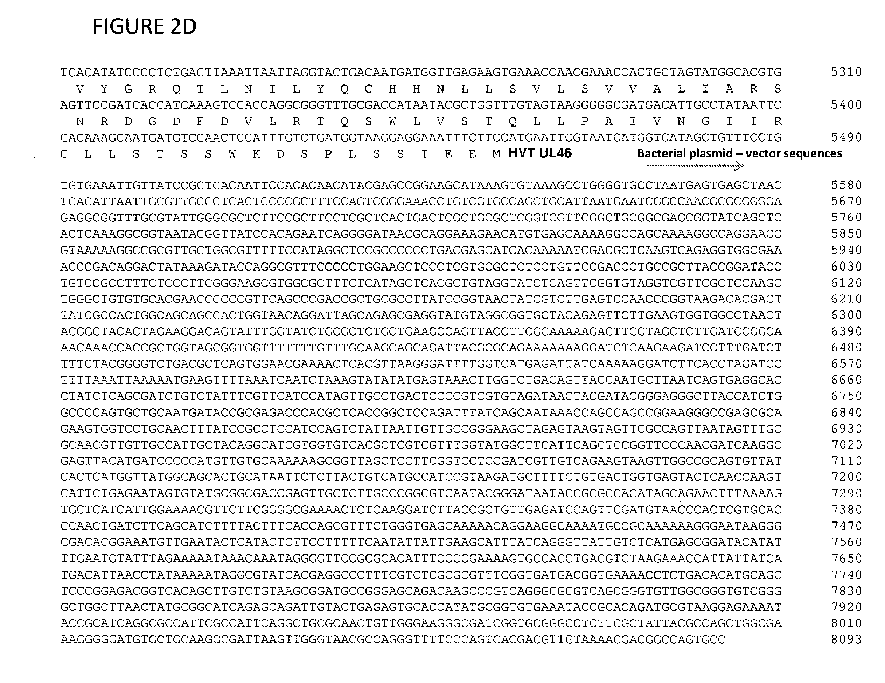

The hemagglutinin gene may be obtained from any subtype or any strain of avian influenza virus. Preferably, the HA gene is obtained from an avian influenza virus of the H5 subtype. More preferably, the HA gene is obtained from avian influenza virus of the H5N1 subtype. Most preferably the HA gene is obtained from influenza virus H5N1 (swan/Hungary/4999/2006) strain. The nucleotide and corresponding amino acid sequences are provided in Figure 2C.

Alternatively, the HA gene is obtained from the avian influenza virus A/Turkey/Wisconsin/68 (H5N9) strain. A nucleotide sequence of the HA gene from the A/Turkey/Wisconsin/68 (H5N9) strain is described in US patent application 2008/0241188. This sequence differs from the published nucleotide sequence of the HA gene of the A/Turkey/Wisconsin/68 (H5N9) strain (M. Garcia et al., 1997, Virus Res. 51: 115-124, GenBank Accession# U79456) by several bases.

According to this preferred embodiment of the present invention, a promoter is operably linked to the HA gene in the viral genome, typically at the 5' region of the HA gene, to control expression of the HA gene and the generation of the HA protein. Preferably, the HA gene is under control of the cytomegalovirus immediate early promoter (CMV promoter), the chicken beta-actin promoter (T.A. Kost et al., 1983, Nucleic Acids Res. 11:8287-8301), or a modified chicken beta-actin promoter (U.S. Pat. No. 6,866,852). A nucleotide sequence of the CMV promoter is described in the literature (M. Boshart et al., 1985, Cell 41: 521-530, GenBank Accession# K03104). Alternatively, the CMV promoter may have a sequence slightly modified from the original sequence as shown in US 2008/0241188.

Also, the region of insertion may be a region in the viral genome which is not essential for virus growth as described above. In other words, a non-essential region may be defined as a region where modification or insertion of a foreign gene does not prevent the virus from replicating successfully in vitro or in vivo. Several non-essential regions in the HVT genome have been reported. As described above, the HA gene and the CMV promoter can be inserted into, but not limited to UL43 (WO 89/01040), US2 (WO 93/25665) or inter-ORF region between UL44 and UL46 (WO 99/18215). Most preferably, the HA gene and the CMV promoter are inserted into the inter-ORF region between UL45 and UL46.

Since the HA protein is a protective antigen of avian influenza virus and the backbone HVT is a live Marek's disease vaccine, the recombinant HVT containing the HA gene in the present invention may be used as a bivalent vaccine against avian influenza and Marek's disease or as a monovalent vaccine against avian influenza. In addition, the vaccine of the present invention can be used in a mixture with any recombinant or non-recombinant viruses such as the MDV serotype 1 or serotype 2 vaccine strains.

The recombinant HVT or MDV expressing one or more different heterologous or homologous peptides of specific pathogens and vaccines according to the invention are particularly useful for vaccinating waterfowl species as described above, particularly of the order of the Anseriformes, including those of the Anatidae family, such as ducks, geese and swans, and of the Anatinae, Tadorminae, and Dendrocygninae subfamilies, susceptible to these pathogens.

As demonstrated in the Examples below, vaccination with such a live vector vaccine is followed by replication of the recombinant HVT within the inoculated host, expressing in vivo the heterologous or homologous peptide or protein in waterfowl cells. The heterologous or homologous immunogenic peptides or proteins then elicited a stable long lasting immunological response. If the antigenic peptide or protein derived from a specific pathogen can stimulate a protective immune response, then the waterfowl inoculated with a recombinant HVT according to the invention will be immune to subsequent infection by that pathogen as well as to infection by MDV. Thus, a heterologous or homologous nucleic acid sequence incorporated into the insertion region of the HVT genome according to the invention may be continuously expressed in vivo, providing a stable, safe and long lasting immunity to a pathogen.

The present invention further relates to the use of the vaccine as described above for immunizing waterfowl species and to method of immunizing waterfowl species by administering an immunologically effective amount of the vaccine according to the invention. The vaccine may be advantageously administered to waterfowl species intradermally, subcutaneously, intramuscularly, orally, *in ovo,* by mucosal administration or via oculo-nasal administration.

The present invention finally relates to vaccination kits for immunizing waterfowl species which comprises an effective amount of the rHVT or MDV vaccine as *descri*bed above and a means for administering said components to said species. Such kit may comprise an injection device filled with the rHVT or rMDV vaccine according to the invention and instructions for intradermic, subcutaneous, intramuscular, or *in ovo* injection. Alternatively, the kit comprises a spray/aerosol or eye drop device filled with the rHVT or rMDV vaccine according to the invention and instructions for oculo-nasal administration, oral or mucosal administration.

The following Examples describe in details the methods and techniques illustrative of the present invention.

### EXAMPLES

### Example 1: The HVT vaccine

Two commercially available HVT (Turkey herpesvirus, MDV serotype 3) vaccines were tested: a cell-associated HVT vaccine (Marek HVT serotype 3 live virus, Wineland) and a freeze-dried HVT vaccine (Bio Marek HVT, Fatro).

### Example 2: Animal and experimental groups

Thirty two day-old male ducks (a sterile, intergenetic cross of Pekin and Muscovy ducks) were used in the experiment. Sixteen ducks were vaccinated with cell-associated vaccine, other sixteen with freeze-dried vaccine. Both vaccines were administered subcutaneously, on day one.

### Example 3: HVT virus detection by PCR

Four ducks from each group were sampled weekly (at 7, 14, 21 and 28 days post-vaccination). Bursa Fabricii, spleen, thymus and feather tip samples were collected at each date (feather tips only from 14 days p.v.) and tested for the presence of HVT vaccine virus. Organ samples were homogenized in sterile PBS to obtain a 10 m/v % suspension. DNA is purified using QIAmp® DNA Mini Kit (QIAGEN, Hilden, Germany) according to the manufacturer's instructions. PCR is performed using primers specific to HVT viruses. A 505 nucleotide long segment of the US3 gene (position 4245-4227) was amplified according to the method described by Handberg et al. (Avian pathol. (2001) pp 243-249). PCR products were analysed on 1.5% agarose gel after ethidium-bromide staining. HVT negative and positive control samples were included in all tests.

### Example 4: Vaccination of waterfowl species

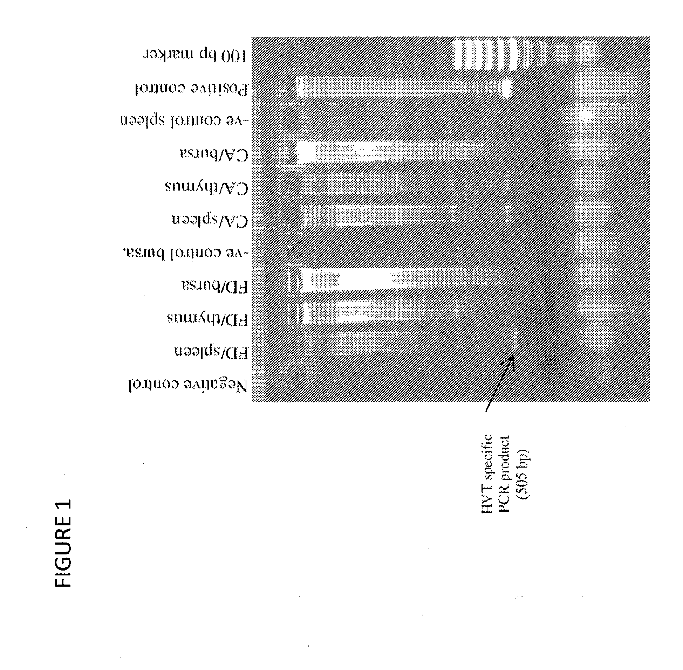

Vaccine virus were detected from 7 (internal organs) or 14 days (feathers) post-inoculation from all birds vaccinated either with the cell-associated or the freeze-dried vaccine. Results are summarized in the following tables and shown in Figure 1.

**Table 1: Detection of HVT vaccine virus by PCR in different organs of ducks after vaccination with cell-associated vaccine**

| Sampling date (day p.v.) | Organs tested | | | |
|---|---|---|---|---|
| | Bursa | Spleen | Thymus | Feather tip |
| 7 | Postive | Positive | Positive | Not tested |
| 14 | Positive | Positive | Positive | Positive |
| 21 | Positive | Positive | Positive | Positive |
| 28 | Positive | Positive | Positive | Positive |

**Table 2: Detection of HVT vaccine virus by PCR in different organs of ducks after vaccination with freeze-dried vaccine**

| Sampling date (day p.v.) | Organs tested | | | |
|---|---|---|---|---|
| | Bursa | Spleen | Thymus | Feather tip |
| 7 | Positive | Positive | Positive | Not tested |
| 14 | Positive | Positive | Positive | Positive |
| 21 | Positive | Positive | Positive | Positive |
| 28 | Positive | Positive | Positive | Positive |

### Example 5: Preparation of HVT-DNA

HVT-DNA was prepared essentially according to the method of Lee et al. (J. of Virol., 7:289-294 (1971)). HVT(FC-126 strain) infected cells (5×10⁷ cells) were cultured in a Leibowitz-L-15/McCoy 5A (1:1) mixed medium in a 16 cm diameter culture dish at 37° C for 4 days. After completion of inclubation, cells were scraped by a scraper and then centrifuged at low speed (2,000 rpm, 5 minutes). After discarding the supernatant, a lysis buffer (0.15 M NaCl, 0.1 M EDTA, 1% SDS, 100 µg/ml proteinase K) in an amount 10 times that of the precipitated infected cells was added. After incubating over night at 37° C, an equal amount of phenol was added to denature proteins, and this procedure was repeated twice for extracting HVT-DNA. The DNA extracted was collected by ethanol precipitation.

### Example 6: Construction of pNZ45/46Sfi

Based on the information on the gCh gene (Coussens et al., J. Virol., 62:2373-2379 (1988)) of the MDV serotype 1 and the flanking EcoRI-BamHI fragment (Japanese Unexamined Patent Publication (Kokai) No. 6-292583) of the BamHI-B fragment, synthetic DNAs (primers 1 to 4) were designed to introduce a Sfil site between the ORF. The above article by Coussens et al. describes UL44 and 45, whereas Japanese Unexamined Patent Publication Asokai) No. 6-292583 describes UL46. Using the primers as described below, PCR was carried out and an amplified product was cloned into pUC18.

From the HVT-FC126-infected CEF, DNA was harvested by the method of Example 5, and 100 ng of the DNA was used as the template. Furthermore, using a "primer set A" consisting of primer 1 (CCCCGAATTC ATGGAAGAAA TTTCC; SEQ ID NO: 2) and primer 2 (CGCGCGCCTT ATTGGCCAAA ACACACCTCT AACGGTTACT; SEQ ID NO: 3) (50 pmol each) and a "primer set B" consisting of primer 3 (GCGCGGCCAA TAAGGCCAA ACACAGTAAC CGTTAGAGGT; SEQ ID NO: 4) and primer 4 (CCCCAAGCTT TCAAGTGATA CTGCGTGA; SEQ ID NO: 5) (50 pmol each), PCR was carried out by a conventional method. The reaction was stopped at the 30th cycle and about 1 µg each of the amplified products was obtained.

Using a mixture of these two PCR products (mixed ratio 1:1, 100 ng of each is mixed) as the template, 30 cycles of PCR (one cycle comprising 45° C. for 1 minute, 60°C for 2 minutes, and 73° C for 3 minutes) were carried out using primer 1 and primer 4, and thereby a Sfil site was introduced in between the ORFs of UL45h and UL46h. The amplified product thus obtained was cleaved with EcoRI and HindII, and the fragment was inserted into the EcoRI-HindIII site of pUC18 using 4 units of T4 ligase by incubating at 16°C for 30 minutes to construct pNZ45/46Sfi.

### Example 7: Construction of pNZ44/45sfi

As in Example 6, using DNA (10 ng) obtained from the HVT-FC126 strain-infected CEF as a template, and using a "primer set C" consisting of primer 1 and primer 5 (GCGCGGCCAA TAAGGCCAAC ATCGGGACGT ACATCAT; SEQ ID NO: 6) (50 pmol each) and a "primer set D" consisting of primer 6 (GCGCGGCCTT ATTGGCCTTA AATACCGCGT TTGGAGTAAA; SEQ ID NO: 7) and primer 4 (50 pmol each), PCR was carried out as in Example 6. Amplified products of about 10 µg each were obtained.
Using a mixture of these two PCR products (mixed ratio 1:1, 100 ng of each is mixed) as a template, PCR is carried out using primer 1 (50 pmol) and primer 4 (50 pmol) as in Example 6, and thereby a Sfi site was introduced in between the ORFs of UL44h (gCh of HSV-1 or gCh (gA) of MDV1) and UL45h.
The product was cleaved with EcoRI and HindIII, and the fragment obtained was inserted into the EcoRI-HindIII site of pUC18 as in Example 6 to construct pNZ44/45Sfi.

### Example 8: Construction of pG1MCSpolyASfi

### (1) Construction of donor plasmid pGTPs

A synthetic DNA (5'-AGCTGCCCCCCCCGGCAAGCTTGCA-3' (SEQ ID NO: 8) was inserted into the Hindlll-Pstl site of pUC18, and this portion was then repaired to double strand using DNA polymerase. Further a synthetic DNA (5'-TCGACATTTTTATGTAC-3'; SEQ ID NO: 9) was inserted into the SalI-KpnI site of the plasmid obtained, which was similarly repaired to double strand with DNA polymerase. Furthermore, an annealed product of two synthetic DNA: 5'-AATTCGGCCGGGGGGGCAGCT-3' (SEQ ID NO: 10) and 5'-GCCCCCCCGGCCG-3' (SEQ ID NO: 11) was introduced to the SacI-EcoRI site of the plasmid. Finally, an about 140 bp DNA fragment obtained by digesting the plasmid pNZ1729R (Yanagida et al., J. Virol., 66:1402-1408 (1992)) with Hindlll and Sacl was inserted to the HindIII-SacI site of this plasmid, to construct a plasmid pGTPs.

### (2) Construction of pGIMCSpolyASfi

PuC18 was cleaved with DraI, and an Xhol linker (manufactured by Takara Shuzo) was inserted therein as in Example 6 to construct pUC18X in which an Xhol site had been introduced.
This pUC18X was cleaved with Hindlll and Pstl, to which synthetic DNA 1 (AGCTTGCCAATAAGGCTGCA; SEQ ID NO: 12) and synthetic DNA 2 (ATGGCCCGCC GGCTGACCGC; SEQ ID NO: 13) were annealed to create a fragment. This was inserted into the above Xhol site using T4 ligase (manufactured by Takara Shuzo) to construct pU18XG.
In order to introduce a poly A addition signal and a Sfil site into the KpnI-EcoRI site of pU18XG, a fragment prepared by annealing a synthetic DNA 3 (GCGGTCAGCC GGCGGGCCAT; SEQ ID NO: 14) and synthetic DNA 4 (GGTAAACTGC AGACTTGGCA GT; SEQ ID NO: 15) was inserted using T4 ligase to construct pUCpolyASfi. Into the RpnI-BamHl site of this pUCpolyASfi, a 36 bp KpnI-BamHI fragment of pGTPs described in Example 4(1) was inserted to construct pMCSpolyASfi. Into the HindIII-PstI site of this pMCSpolyASfi, synthetic DNA 5 (ACTGCCAAGT CTGCAGTTTA CC; SEQ ID NO: 16) was inserted as in Example 6 to construct pGIMCSpolyASfi.

### Example 9: Construction of pRSV and pCMV

An about 600 bp of Nsil-Nhel fragment containing the RSV promoter excised from pBK-RSV by double digestion with Nsil and Nhel was inserted as in Example 6 into the Pstl-Xbal site of PGIMCSpolyASfi that was obtained in Example 8, to construct pRSV.
Similarly, a Nsil-Nhel fragment containing the CMV promoter of pBK-CMV was excised, and was introduced as in Example 6 into the Pstl-Xbal site of pGIMCSpolyASfi that was obtained in Example 8, to construct pCMV.

### Example 10: Construction of pCMV-HN

### (1) Deletion of BglI Sites from the CMV Promoter of pCMV

Since three BglI sites were present in the CMV promoter of pCMV, PCR was carried out as described below to introduce mutation so as to delete the BglI sites. Mutation was carried out in the following manner.
Using pCMV (100 ng) constructed in Example 9 as a template, PCR was carried out under the same conditions as in Example 6 using a primer set E consisting of primer 7 and primer 8 and a primer set F consisting of M13P7 primer (manufactured by Toyoboseki Co., Ltd.) and primer 9. About 10 µg of each of the amplified products was obtained.

Amplified products (100 ng each) obtained using the above 2 primer sets were mixed at a mixed ratio of 1:1. With the mixture as a template, PCR was carried out as in Example 2 using M13P7 primer and primer 8 to obtain about 10 µg of PCA product (1).
Similarly, with pCMV (100 ng) as a template, PCR was carried out as in Example 2 using a primer set E consisting of primer 10 and primer 11 (GGCATAATGC ATGGCGGGCC AT; SEQ ID NO: 17), and primer 12 (ATGGCCCGCC ATGCATTATGCC; SEQ ID NO: 18) and M13P7 primer (manufactured by Toyoboseki Co., Ltd.).
Similarly with the 1:1 mixture of 100 ng each of the products of these two primer sets as a template, PCR was carried out, this time, using primer 10 and M13P8 primer to obtain an about 10 µg of PCR product (2).
Furthermore, the PCR product (1) and the PCR product (2) were mixed. Then using M13P7 primer and M13P8 primer, PCR was carried out as in Example 6 to obtain a CMV promoter in which the BglI sites were deleted.

### (2) Construction of pUCCMV

Also, an about 600 bp of Nsil-Nhel fragment containing the CMV promoter of pBK-CMV was inserted into the Pstl-Xbal site of pUC19 as in Example 6 to construct pUCCMV.

### (3) Construction of pNZ87

### (3-1) Construction of a Plasmid (pNZ76) in which the β-galactosidase Gene was ligated to 7.5K Promoter.

After digesting 10 µg of pMA001 (Shirakawa et al., Gene. 28:127- (1984)) with BamHI, it was extracted with phenol:chloroform (1:1), followed by ethanol precipitation to collect the β-galactosidase gene (about 3.3 kb).
On the other hand, after digesting 0.3 µg of pUC19 with BamHI, it was extracted with phenol:chloroform, followed by ethanol precipitation. The product was ligated to the β-galactosidase gene prepared as above to create a hybrid plasmid pNZ66.
40µg of pUWP-1 (a plasmid containing the promoter of DNA encoding a 7.5KDa peptide of a vaccinia virus WR strain) was digested with Hpall and EcoRI, subjected to 1.5% low-melting point agarose gel electrophoresis (70 V, 6 hours) to separate an about 0.26 kb fragment containing 7.5K promoter, which was then extracted with phenol:chloroform (1:1), and then precipitated with ethanol to collect DNA. The adhesive end of the DNA fragment was blunt-ended using DNA polymerase. After digesting 0.3 µg of pNZ66 with Hincll, it was extracted with phenol:chloroform, followed by ethanol precipitation to collect a fragment, to which was ligated about 0.26 kb of the 7.5K promoter gene mentioned above, and the hybrid plasmid obtained was designated as pNZ76.

### (4) Construction of Hybrid Plasmid pNZ87

Wherein a promoter from a hybrid phage mp10-HN180 and the DNA of the HN gene of NDV has been ligated under the control the promoter pNZ76 was digested with BamHI, which was then subjected to 0.8% agarose gel to collect an about 2.9 kb fragment containing no β-galactosidase gene.
On the other hand, after digesting the hybrid phage mp10-HN180 with BgIII and BamHI, an about 1.8 kb DNA fragment of the NH gene was collected from a 0.8% agarose gel. Both fragments were ligated by ligase. The plasmid obtained was used to transform a competent E. coli TG-1 strain, and a plasmid was extracted using a conventional method. A hybrid plasmid containing the HN gene was detected, which was designated as pNZ87.

### (5) Construction of pNZ87CMV

Then, an about 1.8 kb BamHI-SacI fragment containing the HN gene of NOV was excised from pNZ87 constructed in (3). The fragment was inserted as in Example 6 into the BamHI-Sacl site of pUCCMV to construct pNZ87CMV.
Since pNZ87CMV has BglI sites in the promoter region, the HindIII-BamHI fragment containing the CMV promoter region was replaced with a HindIII-BamHI fragment of the PCR-constructed CMV promoter without the BglI sites described in (1) to construct pCMV-HN (BglI⁻).

### Example 11: Construction of RSV-F

### (1) Construction of pUCRSV-pA

An about 600 bp of Nsil-Nhel fragment containing the RSV promoter of pBK-RSV (STRATAGENE) was inserted into the PstI-XbaI site of pUC19 as in Example 6 to construct pUCRSV. Separately, with pBK-RSV (STRATAGENE, 100 ng) as a template, PCR was carried out as in Example 2 using primer 13 (CGGGAGCTCT AATTGTTTGT G; SEQ ID NO: 19) and primer 14 (CGGGAATTCG CTTACAATTT; SEQ ID NO: 20) (50 pmol each) to obtain a fragment having the pA additional signal of SV40 promoter contained in pBK-RSV. The PCR-amplified fragment was double digested with SacI and EcoRI, and inserted as in Example 6 into the SacI-EcoRI site of pUCRSV to construct pUCRSV-pA.

### (2) Construction of pNZ98

A plasmid XLIII-10H (Virus Research, 7:214-255 (1987)) containing the F gene and HN gene of NDV was used.
Four µg of the plasmid XLIII-10H was digested with Xbal and, after the resulting adhesive end was blunt-ended with DNA polymerase, it was then extracted with phenol:chloroform (1:1), and collected by ethanol precipitation. The collected DNA was digested with BamHI, subjected to a 0.8% agarose gel electrophoresis to collect a fragment containing an about 2.1 kb of complete F gene.
On the other hand, the pNZ76 created as above was double digested with BamHI and Smal to collect an about 3.0 kb BamHI-Smal fragment lacking the lacZ gene portion. The collected fragment and a fragment containing an about 2.1 kb of complete F gene were ligated by ligase to transform a competent E. coli TG1 strain.
A plasmid was prepared by a procedure similar to the described above from a colony grown on an LB agar plate containing 50 µg/ml ampicillin. The plasmid was cleaved with restriction enzymes (BamHI and Smal), the desired clone was confirmed and designated as pNZ98'. This pNZ98' contained the full-length F gene and an about 300 bp 5'-end of the HN gene. In order to remove this portion, pNZ98', was double digested with Smal and KpnI, and an about 4,150 bp Smal-Kpnl fragment was collected by a 0.8% agarose gel electrophoresis. pNZ98' was also double digested with SmaI and AvaII, and an about 650 bp SmaI-AvaII fragment was collected by a 1.5% agarose gel electrophoresis.
These two fragments were mixed, and the resulting adhesive end is blunt-ended with DNA polymerase. The product was then used to transform E. Coli TG1 to obtain a transformant. The transformant was grown on an LB agar medium containing 50 µg/ml ampicillin. From the resulting colony, a plasmid was obtained as mentioned above. The plasmid was digested again with Smal, and the cleaved products were selected to obtain pNZ98. (3) From pNZ98 constructed in above (2), an about 1.8 kb BamHI-SacI fragment containing the F gene of NDV was excised, which was then inserted into the BamHI-SacI site of pUCRSV-pA as described in Example 6 to construct pNZ98RSV3. pNZ98 was also cleaved with Pstl and partially digested with BamHI to obtain a 125 bp fragment.he collected fragment was substituted for a 75 bp PstI-BamHI fragment contained in pNZ98RSV3' to construct pNZ98RSVpA. A 2,892 bp MluI-SacI-cleaved fragment of pRSV constructed in Example 5 and a 2,262 bp MluI-SacI-cleaved fragment of the F gene of NDV of pNZ98RSvpA were ligated to construct pRSV-F.

### Example 12: Construction of pCMV-VP2S

### (1) Construction of pCMV/MCS

Into the BamHI-KpnI site of pCMV-HN (BglI⁻) constructed in Example 10, a 62 bp BamHl-Kpnl fragment of pBluescript SK+ was inserted to construct pCMV/MCS.

### (2) Construction of pCMV/MCSpA

Separately, with pBK-RSV (STRATAGENE) as a template, PCR was carried out as in Example 6 using primer 15 (CCGGGGCCCT AATTGTTTGT G; SEQ ID NO: 21) and primer 16 (CGGGGTACCG CTTACAATTT; SEQ ID NO: 22) to obtain a fragment having the pA additional signal of SV40.
The PCR-amplified fragment was double digested with Apal and Kpnl, and was inserted as in Example 6 into the ApaI-KpnI site of pCMV/MCS to construct pCMV/MCSpA.

### (3) Construction of pCMV-VP2S

Furthermore, RNA was extracted, according to a conventional method, from an IBDV field isolate Okayama strain. The RNA was used to generate cDNA using reverse transcriptase and the cDNA synthesis kit (manufactured by Takara Shuzo).
With this cDNA as a template, primer 17 (GCAAGCTTGC GATGACGAAC CTGC; SEQ ID NO: 23) and primer 18 (GCGTCGACTC ACCTCCTTAG GGCCC; SEQ ID NO: 24) were designed based on a region corresponding to VP2 of the sequence of the gene as set forth in Japanese Unexamined Patent Publication (Kokai) No. 62-503006.
Using these two primers, PCR was carried out as in Example 6 to obtain a region corresponding to the VP2 of IBDV. Separately, pCMV/MCSpA was partially digested with Hindlll, and then partially with SalI, to obtain a 3,687 bp fragment. The fragment of IBDV obtained by the above PCR was double digested with Hindlll and SalI, which was then inserted into a 3,687 bp fragment as in Example 6 to construct pCMV-VP2S.

### Example 13: Construction of pUC18Xlac

The BamHI-SacI fragment of lacZ (Yanagida et al., J. Virol., 66:1402-1408 (1992)) was inserted as in Example 6 into the BamHI-SmaI site of pU18XG constructed in Example 8 to construct pUC18Xlac.

### Example 14: Construction of pNZ45/46RSVlac and pNZ44/45RSVlac

### (1) Construction of pNZ45/46RSVlac

Into the Sfil site of pNZ45/46Sfi constructed in Example 6, the BglI fragment of pRSV containing the RSV promoter constructed in Example 9 was inserted to construct pNZ45/46RSV. Into the Sfi site of pNZ45/46RSV, the BglI fragment of pUC18Xlac containing lacZ constructed in Example 13 was inserted to construct pNZ45/46RSVlac

### (2) Construction of pNZ44/45RSVlac

Similarly, into the Sfil site of pNZ44/45Sfi constructed in Example 7, the BglI fragment of pRSV containing the RSV promoter constructed in Example 9 was inserted to construct pNZ45/46RSV. Into the Sfil site of pNZ44/45RSV, the BglI fragment of pUC18Xlac containing lacZ constructed in Example 13 was inserted to construct pNZ44/45RSVlac.

### Example 15: Construction of pNZ45/46VP2S and pNZ44/45VP2S

### (1) Construction of pNZ45/46VP2S

Into the Sfil site of pNZ45/46RSVlac constructed in Example 14, the BglI fragment of pCMV-VP2S (Okayama) containing the VP2 gene of IBDV constructed in Example 12 was inserted as in Example 6 to construct pNZ45/46VP2S.

### (2) Construction of pNZ44/45VP2S

Similarly, into the Sfil site of pNZ44/45RSVlac constructed in Example 14, the BglI fragment of pCMV-VP2S (Okayama) containing the VP2 gene of IBDV constructed in Example 12 was inserted as in Example 6 to construct pNZ44/45VP2S.

### Example 16: Construction of pNZ45/46HNF and pNZ44/45HNF

### (1) Construction of pNZ45/46HNF

Into the Sfil site of **pNZ45/46RSVlac** constructed in Example 14, the Bgll fragment of pCMV-HN (BglI⁻) containing the HN gene of NDV constructed in Example 10 was inserted as in Example 6 to construct pNZ45/46HN. Furthermore, into the Sfil site of pNZ45/46HN, the BglI fragment of pRSV-F containing the F gene of NDV constructed in Example 11 was inserted to construct pNZ45/46HNF.

### (2) Construction of pNZ44/45HNF

Similarly, into the Sfil site of pNZ45/45RSVlac constructed in Example 14, the BglI fragment of pCMV-SN (BglI⁻) containing the HN gene of NDV constructed in Example 10 was inserted to construct pNZ44/45HN. Furthermore, into the Sfil site of pNZ44/45HN, the BglI fragment of pRSV-F containing the F gene of NDV constructed in Example 11 was inserted to construct pNZ44/45HNF.

### Example 17: Construction of pNZ45/46HNF-VP25 and pNZ44/45VP2S-HNF

### (1) Construction of pNZ45/46HNF-VP2S

Into the Sfil site of pNZ45/46HNF constructed in Example 16, the BglI fragment of pCMV-VP2S containing the VP2 gene of IBDV constructed in Example 12 was inserted to construct pNZ45/46HNF-VP2S.

### (2) Construction of pNZ44/45VP2S-HNF

Into the Sfil site of pNZ44/45VP2S constructed in Example 15, the BglI fragment of pCMV-HN (BglI⁻) containing the RN gene of NDV constructed in Example 10 was inserted to construct pNZ44/45VP2S-HN. Furthermore, into the Sfil site of pNZ44/45VP2S-HN, the BglI fragment of pRSV-F containing the F gene of NDV constructed in Example 11 was inserted to construct pNZ44/45VP2S-HNF.

### Example 18: Purification of Recombinant HVT

A monolayer of CEF that was detached with trypsin is suspended in Saline G (0.14 M sodium chloride, 0.5 mM potassium chloride, 1.1 mM hydrogen disodium phosphate, 1.5 mM dihydrogen sodium phosphate, and 0.5 mM magnesium chloride hexahydrate, 0.011% glucose) to prepare a cell suspension. The cell suspension (2×10⁷ cells) was mixed with 40 µg each of recombinant plasmid pNZ44/45VP2S, pNZ44/45HNF, pNZ45/46VP2S, pNZ45/46HNF, pNZ44/45VP2S-HNF, and pNZ45/46HNF-VP2S, and 100 µg each of the DNA of HVT prepared in Example 9.
After allowing the solution to stand at room temperature for 10 minutes, it was electroporated using the Gene Pulser (manufactured by Bio-Rad) at room temperature under the condition of 3.0 KVcm⁻¹, 0.4 msec, and 25° C. The cells into which the plasmid and the DNA of HVT were introduced and plated on a culture dish of 9 cm in diameter (Falcon), and then were cultured at 37° C for about 4 to 5 days until plaques peculiar to HVT were formed. Cells that formed plaques were scraped with 1% trypsin, and were mixed with CEF cells (2×10⁷ cells) that were similarly scraped with trypsin, which was limiting-diluted in 10 96-well flat-bottomed multi culture plates (manufactured by Falcon). These plates were further cultured at 37° C for about 4 to 5 days until plaques peculiar to HVT were formed in each well. Then, to a half of the wells in each plate, 100 µl/well of the CEF culture medium containing 100 µg/ml of Bluogal (manufactured by Gibco) which was a chromogenic substrate for β-galactosidase and 0.8% agar was added, and the plates were incubated at 37°C for about 4 hours. Thereafter, the number of blue plaques in each well was counted. A plate having the largest number of blue plaques was selected, to which 1% trypsin was added to collect CEF containing recombinant HVT-infected cells. The CEF was mixed with 1 × 10⁶ cells, which was then plated on a 96-well flat-bottomed multi culture plate. Screening Steps (one screening Step comprises one step of passaging from a 96-well flat-bottomed multi culture plate to a 96-well flat-bottomed multi culture plate) were repeated until all wells showed blue plaques, and were repeated until all plaques turned blue when Bluogel was added, and thereby the virus was purified. Purification was normally accomplished by screening about 5 to 10 times. After the infected cells were grown on a culture dish of 9 cm in diameter, the infected cells were further grown on a culture dish of 16 cm in diameter. When the titer of the recombinant HVT is determined, the titer of the recombinant HVT was found to be 1 to 6×10³ TCID₅₀.

### Example 19: Confirmation of expression of an antigen in rHVT-infected cells of waterfowl

On a chamber slide for tissue culture, the above recombinant HVT-infected cells were cultured together with CEF at 37° C. until plaques developed, which were then fixed in cold acetone. In order to detect the expressed antigen, the following were used as primary antibodies. For the detection of β-galactosidase, anti-β-galactosidase rabbit antiserum (polyclonal antibody: manufactured by Organon Teknica H. V.), and for the detection of NDV-NH protein and F protein, NDV vaccine-immunized chicken serum were used each at 500-fold dilution in PBS. For the detection of IBDV-VP2 protein, anti-VP-2 monoclonal antibody GK-5 (Yamaguchi T., et al., Avian Dis., 40:501-509 (1996)) was used at a 100-fold dilution in PBS. As labeled antibody, FITC-labeled anti-chicken IgG antibody, FITC-labeled anti-mouse IgG antibody, and FITC-labeled anti-rabbit IgG antibody (all manufactured by Harlan Sera-Lab Ltd.) were each diluted 100-fold in PBS prior to use.
Each solution containing the above antibody was contacted with cells fixed in cold acetone on the chamber slide, allowed to stand at room temperature at 100% humidity for about 1 hour, and then washed three times in PBS. Then, each solution was allowed to react at room temperature for about 1 hour together with dilutions of FITC-labeled anti-chicken immunoglobulin antibody or anti-mouse IgG. Then each solution was washed three times in PBS, and the reactivity was investigated by examining it under microscope at a fluorescence excitation wavelength (493.5 nm). As a control virus, an HVT parent strain FC-126 was infected, and the cells infected with this parent strain were used as the control cells. The recombinant HVT that incorporated the NDV antigen gene reacted with anti-NDV monoclonal antibody, whereas the recombinant HVT that incorporated IBDV-VP2 gene reacted, with anti-VP2 monoclonal antibody. The result confirmed that each recombinant HVT expressed protein encoded by the inserted gene.

### Example 20: Hemagglutinin gene isolation from avian influenza virus

The amino acid sequence of HA is well known from the reported sequence of A/Turkey/Wisconsin/68 (H5N9) (M. Garcia et al., 1997, Virus Res. 51: 115-124, GenBank Accession# U79456).
The HA amino acid sequence may be derived from the avian influenza virus A/Turkey/Wisconsin/68 (H5N9) strain. The strain wa thus propagated in the allantoic sac of specific pathogen free embryonating eggs. Total genomic RNA from the A/Turkey/Wisconsin/68 virus was extracted using RNEASY MINI KIT (QIAGEN). First-strand cDNA was synthesized with SUPERSCRIPT FIRST-STRAND System for RT-PCR (Invitrogen). Using the resulting cDNA as a template, the HA gene was amplified by polymerase chain reaction (PCR) with PFUULTRA HIGH FIDELITY DNA Polymerase (STRATAGENE) and PCR primers.

### Example 21: Construction of Plasmid

Preferably the CMV promoter, the chicken beta-actin promoter (Bac promoter), and a modified chicken beta-actin promoter (Pec promoter), were used to control expression of the HA gene of the Al virus. First, homology plasmids with the HA gene and one of the promoters were constructed and then recombinant turkey herpes viruses were generated using the homology plasmids.

### (1) Construction of Plasmid pGICMVpA

The CMV promoter was obtained from pBK-CMV (STRATAGENE). Three BglI restriction enzyme sites in the CMV promoter were disrupted for ease of the plasmid construction process by PCR in vitro mutagenesis using four pairs of primers. The CMV promoter fragment was digested with Pstl and Xbal and inserted into Pstl and Xbal digested pUC18polyASfi (U.S. Pat. No. 6,866,852), resulting in pGICMV(-). The SV40 polyA signal was also inserted.

### (2) Construction of Homology Plasmid p45CMVH5Wis68

The CMV promoter and the SV40 polyA signal (940 bp) were excised from pGICMVpA by BglI and ligated into Sfil digested p45/46Sfi (U.S. Pat. No. 6,866,852), resulting in p45/46CMVpA. Then, the HA gene from A/Turkey/Wisconsin/68 (H5N9) was excised from pCR2.1-H5Wis68 using SalI and BamHI. The 1701 bp HA gene was inserted into p45/46CMVpA digested with SalI and BamHI, resulting in p45CMVH5Wis68. The plasmid p45CMVH5Wis68 was used as a homology plasmid to generate recombinant turkey herpes virus.

### (3) Construction of plasmid pGIBacpA2nd

The Bac promoter was obtained by PCR using cellular DNA of CEF cells as a template. PrBac1 and PrBac2' were the primer set used for PCR. An obtained 1.5-kilobase DNA fragment was digested with Pstl and Xbal and inserted into PstI and Xbal digested pUC18polyASfi, resulting in pGIBac2. Then, the SV40 polyA signal obtained PCR using primers PolyA-SalKpn and PolyA-SfiF2 was digested with SalI and Sfil and ligated to pGIBac2 digested with SalI and Sfil resulting in pGIBacpA2nd.

### (4) Construction of homology plasmid p45BaCH5Wis68

The Bac promoter and the SV40 polyA signal (1866 bp) were excised from pGIBacpA2nd by Bgll and ligated into Sfil digested p45/46Sfi, resulting in p45/46BacpA2nd. Then, the HA gene of A/Turkey/Wisconsin/68 (H5N9) was excised from pCR2.1-H5Wis68 using SalI and BamHI was inserted into p45/46BacpA2nd digested with SalI and BamHI, resulting in p45BaCH5Wis68.

### (5) Construction of homology plasmid p45PeCH5Wis68

Construction of the Pec promoter is described in U.S. Pat. No. 6,866,852. The Pec promoter was synthesized by fusing a part of the chicken beta-actin promoter with the enhancer region of the CMV promoter. The Pec promoter was excised from pGIPec (U.S. Pat. No. 6,866,852) with Pstl and BamHI and inserted into Pstl and BamHI digested p45/46BacpA2nd, resulting in p45/46PecpA2nd. Then, the HA gene of A/Turkey/Wisconsin/68 (H5N9) was excised from pCR2.1-H5Wis68 using SalI and BamHI was inserted into p45/46PecpA2nd digested with SalI and BamHI, resulting in p45PeCH5Wis68.

### Example 22: Generation and Isolation of Recombinant Turkey Herpesvirus

Viral DNA of the HVT FC126 strain was prepared as described by Morgan et al. (Avian Diseases, 1990, 34:345-351).10⁷ secondary chicken embryo fibroblast (CEF) cells were suspended in saline G (0.14 M NaCl, 0.5 mM KCI, 1.1 mM Na2HP04, 1.5 mM NaH2PO4, 0.5 mM MgCl2, and 0.011% glucose) and co-transfected with HVT viral DNA and 5 to 25 µg of the homology plasmid, p45CMVH5Wis68, p45BaCH5Wis68, or p45PeCH5Wis68 by electroporation. Electroporation was performed using BIO-RAD GENE PULSER. Transfected cells were incubated for 10 minutes at room temperature and transferred to wells of 96-well plates. After incubating at 37° C for 7 days in 4-5% CO₂, or until the plaques became visible, the cells are detached from the plates by trypsinization, transferred equally to two 96-well plates with secondary CEF and incubated for 3 to 4 days until plaques were observed. Screening is conducted by the black plaque assay, staining only plaques expressing HA protein. Briefly, one of the two plates was fixed with methanol:acetone mixture (1:2) and incubated with chicken anti-HA antiserum. Next, incubated with biotinylated anti-chicken IgG antibody (VECTOR LABORATORIES, Cat# BA-9010) and then with VECTASTAIN ABC-AP kit (Vector Laboratories, Cat# AK-5000), plaques expressing HA protein were stained by addition of BCIP/NBT solution (BIO-RAD LABORATORIES, Cat# 170-6539 and 170-6532). Wells containing stained recombinant plaques were identified and cells from the corresponding wells on the other 96-well plate were trypsinized. The cells were then diluted in fresh secondary CEF cells and transferred to 96-well plates to complete the first round of purification.
The purification procedure was repeated until all plaques were stained positively in the black plaque assay. Purified recombinant virus with the HA gene under the CMV promoter was designated as rHVT/CMVH5Wis68. Recombinant viruses with the Bac promoter or the Pec promoter were designated as rHVT/BaCH5Wis68 and rHVT/PeCH5Wis68, respectively.

### Example 23: Preparation of the rHVT (serotype 3) vectored Avian Influenza virus (H5 subtype) vaccine

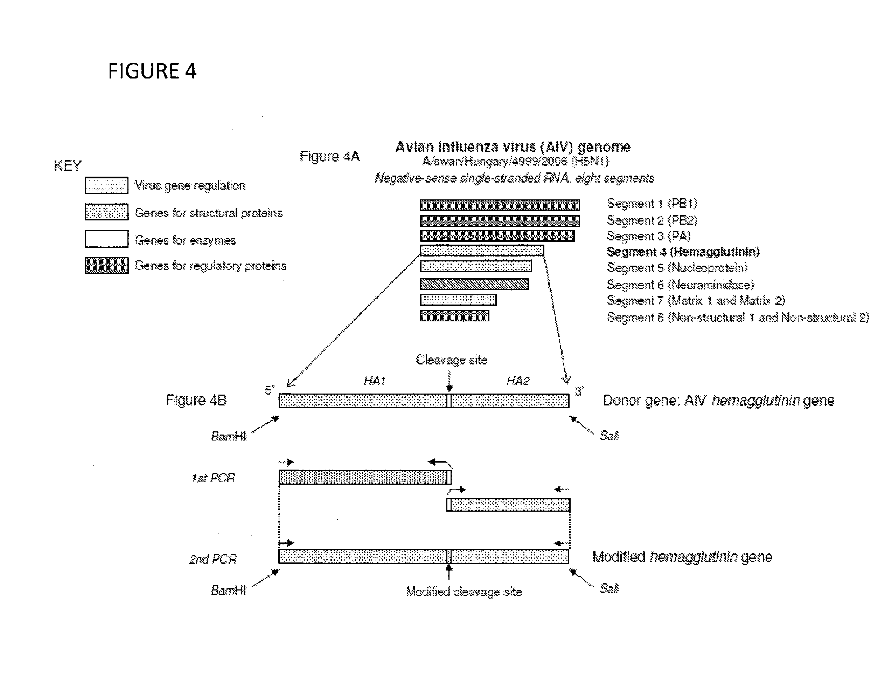

The rHVT (serotype 3) vectored Avian Influenza virus (H5 subtype) vaccine which was designated rHVT/AI-H5 contained a turkey herpes virus (HVT) serotype 3 backbone or vector component and expressed the hemagglutinin (HA) protein of avian influenza virus (AIV) H5 subtype. The HVT parent strain, FC126, has been used commercially to vaccinate ducks. HVT is classified in the Biosafety Level 1 category.
*In ovo* to embryos at 18 days of incubation and subcutaneous to day of age.
The AIV H5 subtype HA gene was placed between two open reading frames in the HVT genome. Attenuation of the HVT vaccine strain has not been observed.

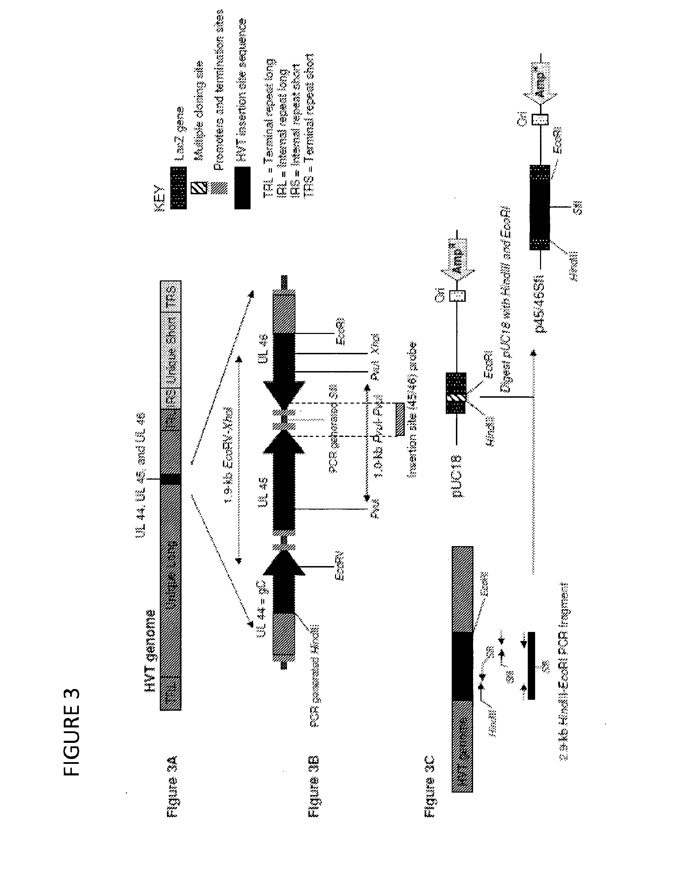

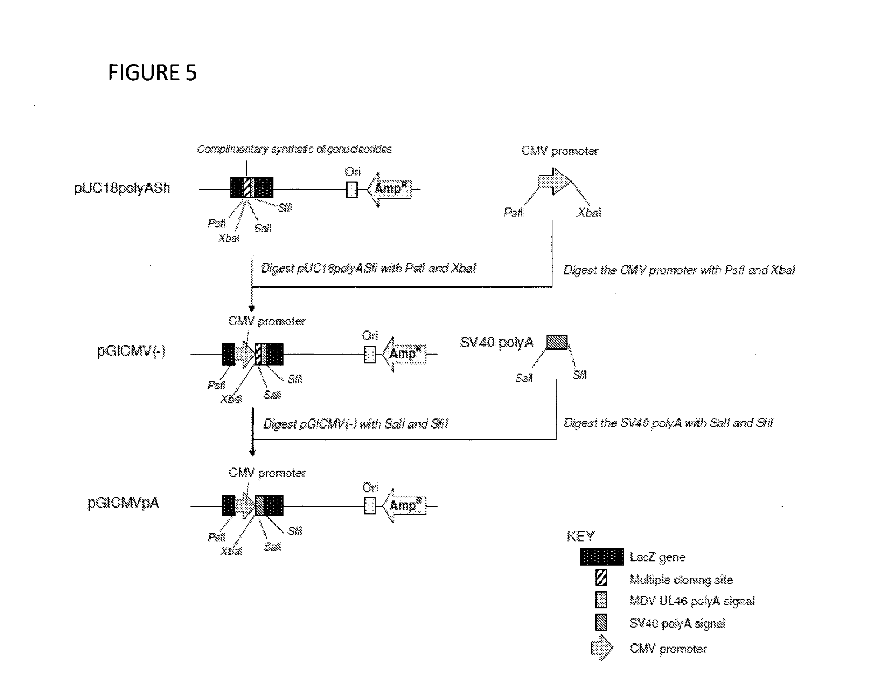

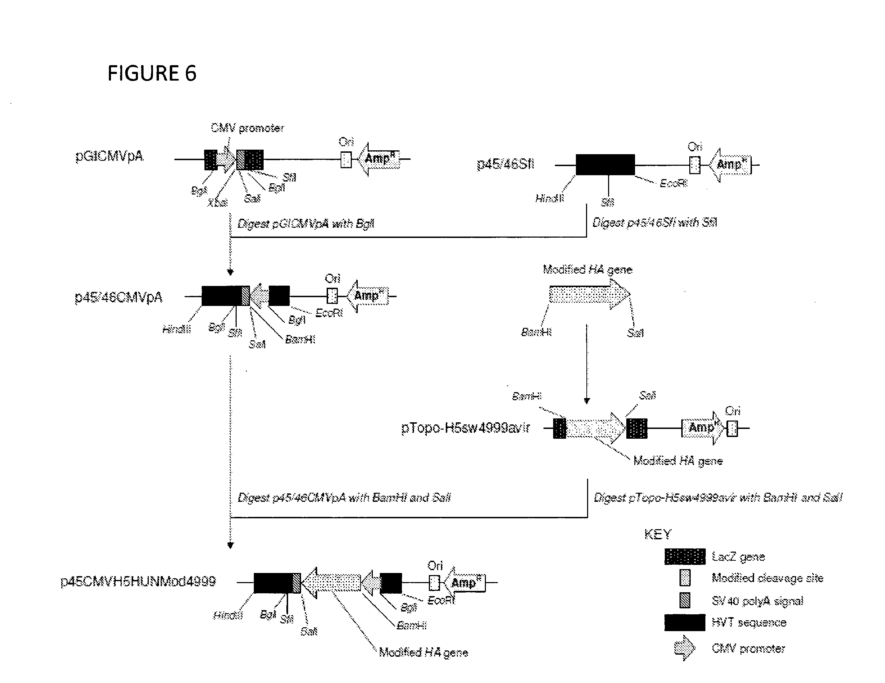

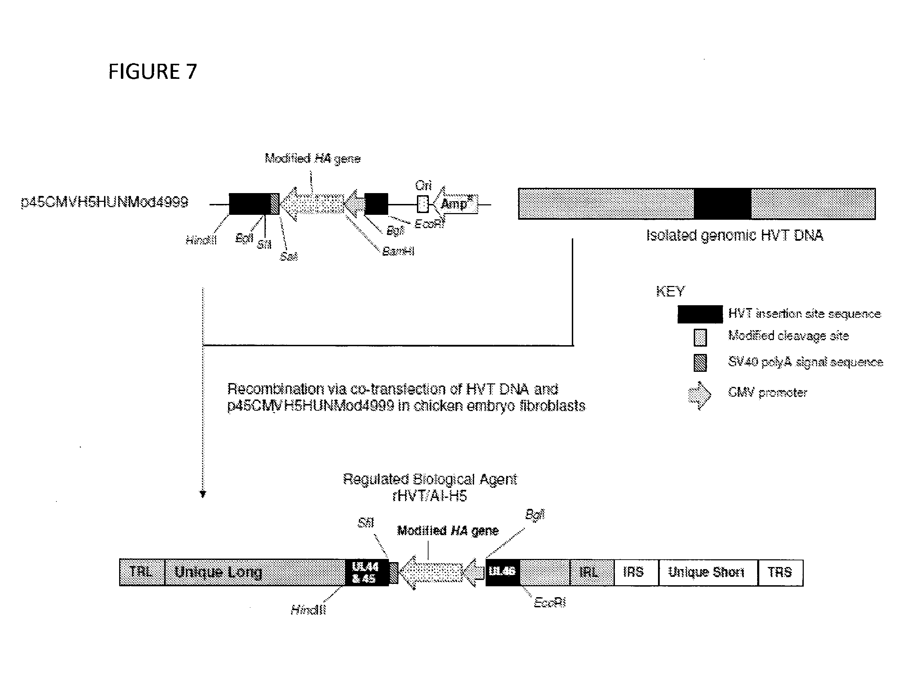

Figures 3-8 provide a complete flow diagram. Polymerase chain reaction (PCR) generated *Sfi*I site between the stop codons of unique long (UL) 45 and UL 46 (Figure 3). The flanking regions of the insertion site were the non-coding regions between UL 45 and UL 46 of the HVT genome. The UL 45 and UL 46 have typical eukaryotic promoters and polyadenylation signals. This insertion site had previously been used in two of our HVT recombinant products, (1) Bursal Disease-Marek's Disease Vaccine, Serotype 3, Live Marek's Disease Vector, and (2) Marek's Disease-Newcastle Disease Vaccine, Serotype 3, Live Marek's Disease Vector.
A portion of UL 44, the entire UL 45 and a portion of UL 46 had been isolated on a 1.9-kilobase (kb) *Eco*RV-*Xho*I fragment of the HVT genome (Figure 3B). In a Southern blot, a probe designed to bind to sequences flanking the insertion site anneals to a 1.0-kb *Pvu*I-*Pvu*I fragment of the HVT genome (Figure 3B).
The description of each Donor biological agent is shown in Figures 4-6. A promoter that controls Donor gene expression was the Cytomegalovirus (CMV) immediate early (IE) promoter (Boshart M. et al., 1985 Cell. 41: 521-530). The SV40 polyadenylation signal (Griffin, DNA Tumor viruses, J. Tooze, ed. Cold Spring Harbor Laboratory Press, Cold Spring Harbor, NY. pp. 843-913) was added to 3' terminus of inserted Donor DNA sequence. The multiple cloning sites were derived from pUC18.
The inserted Donor DNA sequence was a fragment amplified by reverse transcriptase-polymerase chain reaction (RT-PCR) from negative-sense, single-stranded RNA genome of AIV H5 subtype strain (Figure 4). The size of the inserted sequence was 1695 base pairs (bp).
The *HA* gene was isolated from influenza virus A H5 subtype strain, A/swan/Hungary/4999/2006 (H5N1). A/swan/Hungary/4999/2006 (H5N1) is a highly pathogenic strain which was isolated from a swan in Hungary. There is no known previous use of this isolate. Cloned *HA* gene was obtained from Hungary in accordance with United States Veterinary Permit Number 101039. The cleavage site of the *HA* gene from A/swan/Hungary/4999/2006 (H5N1) was altered to typical cleavage site sequence of low pathogenic avian influenza strains. The AIV *HA* gene was used in a USDA-licensed vaccine, which is a fowlpox vectored avian influenza vaccine.
The Donor gene was the *HA* gene of AIV (H5 serotype). The gene was isolated from complementary DNA (cDNA) by converting the negative-sense, single-stranded RNA genome of AIV to cDNA using reverse transcriptase (Figure 4). The *HA* gene was amplified by polymerase chain reaction (PCR) using forward and reverse PCR primers, producing 1.7-kb fragment (Figure 4). These PCR primers annealed to the start and stop sequences of the *HA* gene and each primer also contained a sequence for a restriction enzyme, *Bam*HI or *Sal*I at the 5' ends. Modification of the cleavage site was conducted by PCR using PCR primers carrying desired sequences.

### (1) Construction and Characterization of the rHVT/AI-H5

Diagrams for the construction of the Regulated Biological Agent are provided as described below.
The HVT (Backbone Biological Agent) genome was shown in Figure 3. The AIV (Donor Biological Agent) genome and the *HA* gene were shown in Figure 4. Plasmids used for construction of the rHVT/AI-H5 were described in Figures 3 through 6. *E*. *coli* TOP10, TG-1 or JM109 were used to transform and amplify plasmids. Chicken embryo fibroblasts (CEF) were used as the host cell for recombination of the homology plasmid and HVT genomic DNA.
After transfection, virus successfully grown in CEF was separated by limiting dilutions. Wells containing plaques were expanded in duplicate and screened for expression of the *HA* gene. The recombinant virus was passed seven times to isolate a clone of rHVT/AI-H5 and five times to expand the clone that was designated as master seed virus (MSV). Gene insertion was characterized by genetic detection as described above. The AIV HA product was detected by Western blotting of CEF infected with rHVT/AI-H5, using an antibody to the HA protein of AIV.

### (2) Physical Characterization of the rHVT/AI-H5

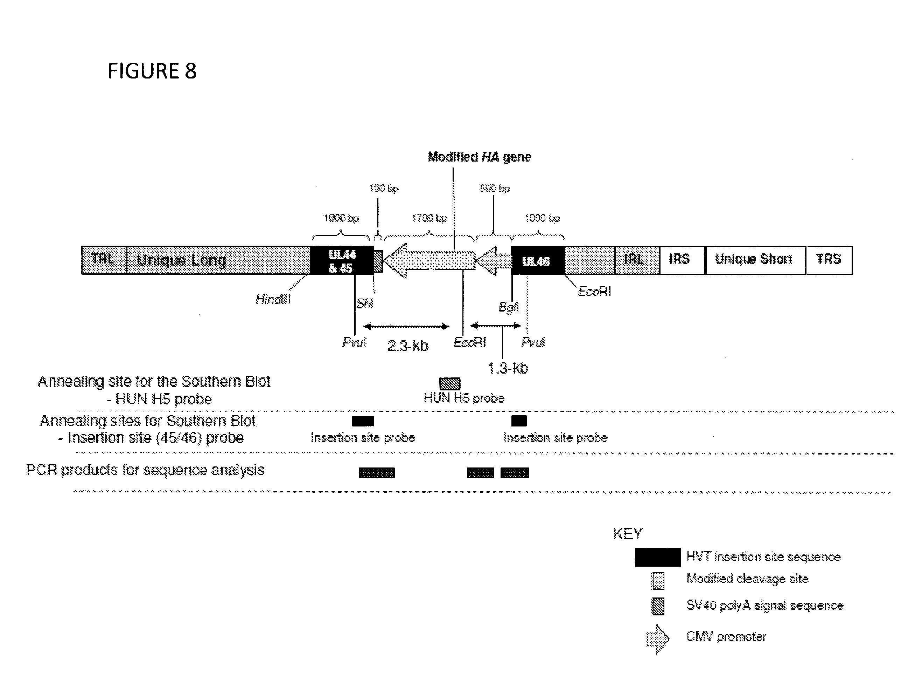

Southern blot analysis and PCR and sequencing of important regions were conducted to characterize the physical map of the RBA. Two Southern blotting schemes are provided in Figure 8 as well as three PCR schemes to amplify three regions. The sequences of these PCR products were analyzed.
A 2.3-kb *Pvu*I-*Eco*RI fragment and a 1.3-kb *Eco*RI-*Pvu*I fragment define the insert and HVT sequences flanking the insertion. In one Southern blot, a probe designed to hybridize to the *HA* gene annealed to the 2.3-kb *Pvu*I-*Eco*RI fragment (Figure 8). In the second Southern blot, a probe designed to hybridize to sequences flanking the insertion site, referred to as the insertion site probe, annealed to both the 2.3-kb *Pvu*I-*Eco*RI fragment and the 1.3-kb *Eco*RI-*Pvu*I fragment (Figure 8). The insertion site probe, which was also called 45/46 probe, annealed to the 1.0-kb *Pvu*I-*Pvu*I fragment in the HVT parent genome (Figure 3B). The two Southern blots and the three PCR schemes described in Figure 8 were used to test MSV n and n+5.
Because the *HA* gene did not provide any new virulence factors to the HVT backbone and rHVT/AI-H5 was expected to be biologically the same as the parent virus, the recommended NIH/CDC biosafety level is the same as the parent virus (Biosafety Level 1). Genetic motifs were the CMV promoter and the SV40 polyadenylation signal. The CMV promoter and the SV40 poly A addition sites have been used extensively for production of safe expression vectors. Bacterial plasmid components were derived from well-defined pUC-based vectors. Vectors (such as pUC) and host strains (such as *E. coli* TOP10, TG-1, and JM109) were considered to be Biosafety Level 1 pathogens.

### (3) Phenotypic Characteristics of the rHVT/AI-H5

The cloning site between UL 45 and UL 46 was thought to be non-essential for HVT replication since the RBA replicates similar to the HVT parent strain *in vitro* and *in vivo.* This insertion site has previously been used in two of the HVT recombinant products, (1) Bursal Disease-Marek's Disease Vaccine, Serotype 3, Live Marek's Disease Vector and (2) Marek's Disease-Newcastle Disease Vaccine, Serotype 3, Live Marek's Disease Vector. The rHVT/AI-H5 expressed the *HA* gene of AIV. Expression of the HA protein were demonstrated by black plaque assay and western blot analysis using anti-HA antibodies.

### Example 24: Effect of waterfowl rHVT vaccination against NDV and Avian Influenza virus

An experiment on the vaccination effect was carried out in order to determine the effects of the recombinant HVT vaccine obtained in Example 18. Ten test ducks and ten test geese per group were inoculated with a recombinant HVT (rHVT-ND or rHVTAI) according to the invention. A commercial inactivated combined ND+AI vaccine was inoculated to the positive control group (hatch-mates of the test ducks and geese), while the negative control birds get no vaccination.
When test ducks and geese hatch, each recombinant HVT was subcutaneously inoculated on the back of the neck of the ducks and geese using a 26 G needle to give 5x10³ PFU₅₀. The commercial inactivated vaccine used for the positive controls was inoculated to hatch-mate geese or ducks by subcutaneous route.
From 2 to 6 weeks post-vaccination each bird was blood sampled at weekly intervals and the serum therefrom was tested to detect antibodies that suppress hemagglutination activity (haemagglutination inhibition, HI) of NDV and AIV which one was the relevant. The determination of HI antibodies was carried out according to internationally recognized test method.
In a separate trial 20 test ducks were inoculated on the back of the neck using a 26 G needle to give 5x10³ EID₅₀ of the recombinant HVT-AI vaccine. At 4 weeks after inoculation, the test ducks along with ten unvaccinated control ducks were challenged with a highly pathogenic avian influenza (HPAI) H5N1 subtype virus strain by administering it at 10⁶ EID₅₀ by the oral-nasal route. The clinical signs indicative of HPAI virus infection and the mortality of ducks during a 2 weeks observation period were recorded to determine the effects using the survival rate as an index. Before challenging the ducks with the HPAI H5N1 virus, blood was drawn from each duck and the serum therefrom was tested to detect antibodies that suppress hemagglutination activity of homologue H5N1 avian influenza HA antigen.

### Example 25: Efficacy of a recombinant rHVT-AI/H5 vaccine in Muscovy ducks against challenge with the H5N1 highly pathogenic Avian Influenza clade 2 A/Duck/Hungary/1180/2006

The experiment was carried out to evaluate the protection afforded by a recombinant rHVT-AI/H5 vaccine administered to day old commercial Muscovy ducks (♀ only) against the highly pathogenic H5N1 Al clade 2 Hungary virus strain *A*/*Duck*/*Hungary*/*1180*/*2006.* Group I of this test consisted of test birds, inoculated at the day of hatch (Day 0) with the rHVT-cH5 vaccine ("rHVT-cH5(Hun06) mod. cell-associated frozen" vaccine) at a dose of 3000 PFU in 0.2 ml per duck subcutaneously (in the neck). Group II was the control non vaccinated group. Both the groups were placed in separate BSL3 isolators. The ducks from both groups were then challenged by oculo-nasal route with 10⁶ EID₅₀ of H5N1 clade 2 *A*/*Duck*/*1180*/*2006* HPAI virus strain at 32 days of age. Before challenge (on Day 29), blood samples for serology were taken from each animal from both groups. Blood samples were taken at 2 time points from the vaccinated and unvaccinated groups: before challenge and at the end of the experiment (2 weeks after challenge). Haemagglutination inhibition tests were performed according to standard procedures. Titres > 2³ were considered as positive Post challenge, the animals were observed daily for clinical symptoms in each group. The blood samples of the ducks which survived the challenge were taken on day 46 before sacrificing the ducks.

**Table 3**

| Groups | Number of ducks | Age of vaccination | Vaccine | Challenge |
|---|---|---|---|---|
| I | 11 | 0 day | rHVT-cH5(Hun06) mod. | 11 ducks, D32 |
| | | | | H5N1 Hungary 2006 |
| II | 11 | - | - | 11 ducks, D32 |
| | | | | H5N1 Hungary 2006 |

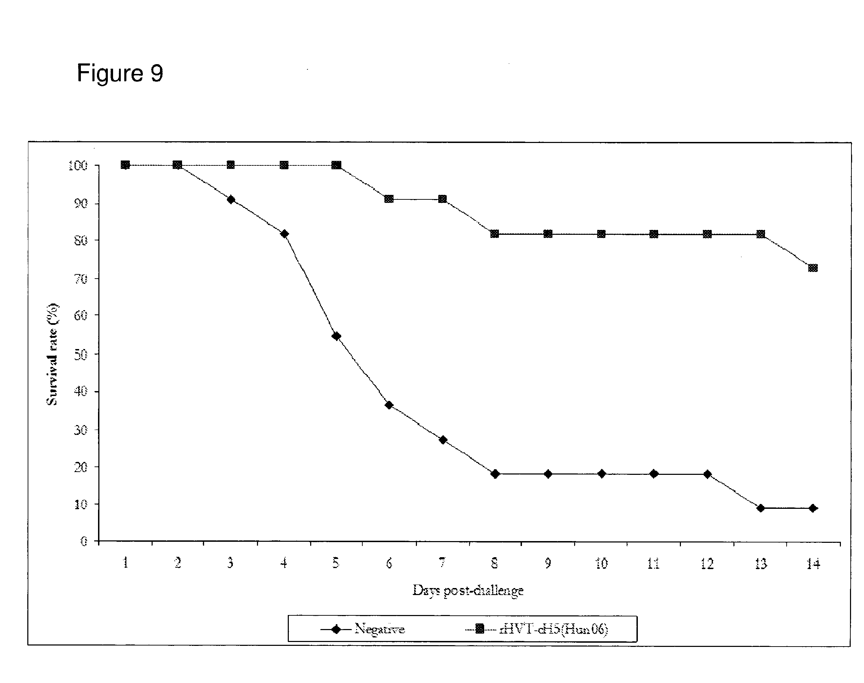

The results were represented in Figure 9. In the control group, clinical symptoms were observed from day 2 post inoculation starting with two ducks (-B and -YY). Mortality began at day 3 post inoculation and 9 out of 11 ducks were dead by day 8 post inoculation. One bird died at the end of experiment while one duck recovered. Whilst in the test group clinical symptoms were observed first on day 4 post inoculation on two ducks (WW and N) which died at days 6 and 8 post inoculation. Two ducks were sick (BB and R) at the end of experiment but only one died(R).

The results of the immune response to vaccination are given below in Tables 4 and 5.

**Table 4**

| Times | Groups | | p-value | Comparison |
|---|---|---|---|---|
| | Negative | rHVT-cH5(Hun06) | | |
| | (group II) | (group I) | | |
| | H5N1 clade 2 *A*/*Swan*/*Hungary*/*4571*/*2006* antigen | | | |
| Before challenge | < 2³ | 6.36 ± 1.91^{A} | 0.000 | I > II |
| After challenge | 7.00 | 8.00 ± 0.93 | N.T. | |
| p-value | N.T. | 0.026 | | |
| Comparison | | After > Before | | |

| | | | | |
|---|---|---|---|---|
| N.T. = not tested because of not data enough in negative group (only one duck survived to the challenge). | | | | |

**Table 5**

| Vaccinated ducks | Days post-infection | | |
|---|---|---|---|
| | H5N1 clade 2 *A*/*Swan*/*Hungary*/*4571*/*2006* antigen | Morbidity | Mortality |
| B | 7 | - | - |
| BB | 7 | - | - |
| G | 8 | - | - |
| GG | 7 | - | - |
| R | 6 | 13 | 14 |
| RR | 7 | - | - |
| W | 8 | - | - |
| WW | 7 | 4 | 8 |
| Y | 6 | - | - |
| YY | 6 | - | - |
| N | <2 | 4 | 6 |

After challenge with H5N2 clade 2 *A*/*Swan*/*Hungary*/*4571*/*2006* HA antigen, all non-vaccinated ducks were HI negative at 4 weeks of age (before the challenge), while among the rHVT-cH5 vaccinated animals, except 1 bird, all animals developed HI antibodies to this antigen. Two weeks after the challenge, all animals were positive in both groups. A booster effect due to challenge was observed and reached significant level (*P* < *0.05*) in the vaccinated group (negative group was not analysed statistically since only one animal survived to the challenge).
Hence non-vaccinated ducks showed 100% morbidity and 91 % mortality within 2 weeks when challenged with H5N1 clade 2 *A*/*Duck*/*Hungary*/*1180*/*2006* strain at 32 days of age. Among this group, 18 % showed clinical signs on 2^{nd} day post inoculation and all ducks were sick on 3^{rd} day post inoculation. First dead animals were observed on 3^{rd} day post inoculation and the peak of mortality was noted between the 4^{th} and 7^{th} days post inoculation. At the end of observation period, 9 % of sick non-vaccinated ducks recovered. 72 % of ducks vaccinated with rHVT-cH5 cell-associated frozen vaccine at hatch survived when challenged 4 weeks after vaccination, with first dead birds were seen on 6th day post inoculation. In the vaccinated group, 18 % died between 6^{th} and 8^{th} day post inoculation and 9 % on 14^{th} day post inoculation. Clinical signs were observed from 4^{th} day post inoculation on animals which died earlier and only at the end of the observation period for the one which died on 14^{th} day post inoculation.
These results confirmed the superior efficacy of the recombinant "rHVT-cH5(Hun06) mod. cell-associated frozen" vaccine administered subcutaneously to day-old ducks, against the clinical symptoms and mortality caused by HPAI H5N1 clade 2 virus challenge.
Also, using HA antigen prepared from AIV strain (*A*/*Swan*/*Hungary*/*4571*/*2006)* donating the insert to the recombinant vaccine the measured serological response to vaccination was reasonably high (6.36 ± 1.91 log2). Following challenge with H5N1 clade 2 *A*/*Duck*/*Hungary*/*1180*/*2006* strain, a boost effect was observed.

## Claims

1. A recombinant Marek's disease virus (rMDV) comprising, inserted into the viral genome, at least one heterologous or homologous nucleotide sequence encoding at least one antigenic peptide, for use in vaccinating waterfowl.

2. The rMDV for use of claim 1, wherein the recombinant Marek's disease virus is a serotype 1, serotype 2, or serotype 3 virus, preferably a serotype 3 recombinant herpes virus of turkeys (rHVT).

3. The rMDV for use according to any one of the preceding claims, wherein the nucleotide sequence encodes an antigenic peptide from avian influenza virus, avian paramyxovirus type 1 (Newcastle disease virus, NDV), avian metapneumovirus, Marek's disease virus, Gumboro disease virus (infectious bursal disease virus: IBDV), goose and Muscovy duck parvoviruses, duck virus enteritis herpes virus, goose herpesvirus, duck hepatitis virus type 1, 2 and 3, goose haemorrhagic polyomavirus, duck and goose adenoviruses, goose and duck circoviruses, West Nile virus, goose and Muscovy duck reoviruses, Escherichia coli, Salmonella species, Pasteurella multocida, Riemerella anatipestifer, Ornithobacterium rhinotracheale, Mycoplasma gallisepticum, Mycoplasma synoviae, a Mycoplasmas microorganisms capable of infecting waterfowl species or coccidian.

4. A rMDV, preferably a rHVT, comprising a heterologous nucleic acid sequence that encodes influenza haemagglutinin (HA), for use as a veterinary drug or vaccine for protecting waterfowl against influenza.

5. The rMDV for use according to any one of the preceding claims, wherein the rMDV further comprises one or more additional homologous or heterologous nucleotide sequences.

6. The rMDV for use according to any one of the preceding claims, wherein the rMDV further comprises another nucleotide sequence which encodes an immunomodulator.

7. The rMDV for use of claim 6, wherein the immunomodulator is a lymphokine, an interferon, or an avian cytokine.

8. The rMDV for use according to any one of the preceding claims, wherein the nucleotide sequence is inserted into a non coding region or inter-ORF region of the backbone virus genome.

9. The rMDV for use according to any one of the preceding claims, wherein the nucleotide sequence is inserted into an untranslated region of the viral genome.

10. The rMDV for use according to any one of the preceding claims, wherein the nucleotide sequence is inserted into an untranslated region located between UL44 and UL45, between UL45 and UL46, between UL41 and UL42, between UL40 and UL41, a region located downstream of the gB gene, between UL53 and UL54, or between UL36 and UL37.

11. The rMDV for use according to any one of the preceding claims, wherein the nucleotide sequence is under control of an endogenous or a heterologous promoter.

12. The rMDV for use according to any one of the preceding claims, wherein the antigenic peptide is expressed in cells of bursa, spleen, thymus and feather follicles of the vaccinated waterfowl species.

13. The rMDV for use according to any one of the preceding claims, wherein said waterfowl species are vaccinated at least one day of age.

14. The rMDV for use according to any one of the preceding claims, which is administered via intradermic injection, subcutaneous injection, intramuscular injection, *in ovo* injection, oral administration, mucosal administration, or via oculo-nasal administration.

15. The rMDV for use according to any one of the preceding claims, which is a multivalent vaccine comprising a mixture of at least two vaccines, one of which is the recombinant MDV as defined in any one of the preceding claims.

16. The rMDV for use according to any one of the preceding claims, which is a polyvalent vaccine comprising two or more recombinant MDV expressing a single or different immunizing protein antigen.

17. A vaccine for use in immunizing waterfowl species, which comprises an effective immunizing amount of a recombinant Marek's disease virus (rMDV) comprising, inserted into the viral genome, at least one heterologous or homologous nucleotide sequence encoding and expressing in cells of waterfowl species at least one antigenic peptide.

## Patentansprüche

1. Ein rekombinantes Mareksche Krankheit-Virus (rMDV) umfassend, eingebaut in das virale Genom, mindestens eine heterologe oder homologe Nukleotidsequenz, die für mindestens ein Peptidantigen kodiert, zur Verwendung bei der Vakzinierung von Wasservögeln.

2. Das rMDV zur Verwendung nach Anspruch 1, wobei das rekombinante Mareksche Krankheit-Virus ein Serotyp 1-, Serotyp 2- oder Serotyp 3-Virus ist, vorzugsweise ein rekombinantes Serotyp 3 Herpesvirus (rHVT) des Truthahns ist.

3. Das rMDV zur Verwendung nach einem der vorhergehenden Ansprüche, wobei die Nukleotidsequenz für ein Peptidantigen aus dem Influenzavirus der Vögel, aus dem Paramyxovirus Typ 1 der Vögel (Newcastle Krankheit-Virus, NDV), aus dem Metapneumovirus der Vögel, aus dem Mareksche Krankheit-Virus, aus dem Gumboro-Krankheit-Virus (Ansteckende Bursakrankheit-Virus: IBDV), aus den Parvoviren der Gänse und Moschusenten, aus dem Virusenteritis-Herpesvirus der Enten, aus dem Herpesvirus der Gänse, aus dem Hepatitisvirus der Enten des Typs 1, 2 und 3, aus dem hämorrhagischen Polyomavirus der Gänse. aus den Adenoviren der Enten und Gänse, aus den Circoviren der Enten und Gänse, aus dem West-Nil-Virus, aus den Reoviren der Gänse und Moschusenten, aus Escherichia coli, aus Salmonella Arten, aus Pasteurella multocida, aus Riemerella anatipestifer, aus Ornithobacterium rhinotracheale; aus Mycoplasma gallisepticum, aus Mycoplasma synoviae, aus einem Mycoplasma-Mikroorganismus, der Wasservogelarten infizieren kann, oder aus Coccidien kodiert.

4. Ein rMDV, vorzugsweise ein rHVT, umfassend eine heterologe Nukleinsäuresequenz, die für ein Influenza-Hämagglutinin (HA) kodiert, zur Verwendung als Veterinärwirkstoff oder Vakzin zum Schutz von Wasservögeln gegen Influenza.

5. Das rMDV zur Verwendung nach einem der vorhergehenden Ansprüche, wobei das rMDV ferner eine oder mehrere zusätzliche homologe oder heterologe Nukleotidsequenzen umfasst.

6. Das rMDV zur Verwendung nach einem der vorhergehenden Ansprüche, wobei das rMDV ferner eine weitere Nukleotidsequenz umfasst, die für einen Immunmodulator kodiert.

7. Das rMDV zur Verwendung nach Anspruch 6, wobei der Immunmodulator ein Lymphokin, ein Interferon oder ein Vogelzytokin ist.

8. Das rMDV zur Verwendung gemäß einem der vorhergehenden Ansprüche, wobei die Nukleotidsequenz in einen nicht-kodierenden Bereich oder einen inter-ORF-Bereich des Virusgenomrückgrats eingesetzt ist.

9. Das rMDV zur Verwendung gemäß einem der vorhergehenden Ansprüche, wobei die Nukleotidsequenz in einen nicht-translatierten Bereich des viralen Genoms eingesetzt ist.

10. Das rMDV zur Verwendung gemäß einem der vorhergehenden Ansprüche, wobei die Nukleotidsequenz in einen nicht-translatierten Bereich, der sich zwischen UL44 und UL45, zwischen UL45 und UL46, zwischen UL41 und UL42, zwischen UL40 und UL41 befindet, in einen Bereich der sich stromabwärts des gB-Gens befindet, zwischen UL53 und UL54, oder zwischen UL36 und UL37 eingesetzt ist.

11. Das rMDV zur Verwendung gemäß einem der vorhergehenden Ansprüche, wobei die Nukleotidsequenz unter Kontrolle eines endogenen oder eines heterologen Promotors steht.

12. Das rMDV zur Verwendung gemäß einem der vorhergehenden Ansprüche, wobei das Peptidantigen in Zellen der Bursa, der Milz, des Thymus und der Federfollikel der geimpften Wasservogelarten exprimiert wird.

13. Das rMDV zur Verwendung gemäß einem der vorhergehenden Ansprüche, wobei die Wasservogelarten mindestens vakziniert werden, wenn sie einen Tag alt sind.

14. Das rMDV zur Verwendung gemäß einem der vorhergehenden Ansprüche, das über intradermale Injektion, subkutane Injektion, intramuskuläre Injektion, *in ovo* Injektion, orale Verabreichung, mukosale Verabreichung oder über oculo-nasale Verabreichung verabreicht wird. 2

15. Das rMDV zur Verwendung gemäß einem der vorhergehenden Ansprüche, das ein multivalentes Vakzin ist, das ein Gemisch mindestens zweier Vakzine umfasst, von denen eines das wie in einem der vorhergehenden Ansprüche definierte, rekombinante MDV ist.

16. Das rMDV zur Verwendung gemäß einem der vorhergehenden Ansprüche, das ein polyvalentes Vakzin ist, das zwei oder mehr rekombinante MDV umfasst, die ein einzelnes oder unterschiedliche immunisierende Proteinantigene exprimieren.

17. Ein Vakzin zur Verwendung bei der Immunisierung von Wasservogelarten, das eine wirksame Immunisierungsmenge an rekombinantem Mareksche Krankheit-Virus (rMDV) umfasst, umfassend, eingesetzt in das virale Genom, mindestens eine heterologe oder homologe Nukleotidsequenz, die für mindestens ein Peptidantigen kodiert und dieses in Zellen von Wasservogelarten exprimiert.

## Revendications

1. Virus de la Maladie de Marek recombinant (rMDV) comprenant, inséré dans le génome viral, au moins une séquence nucléotidique hétérologue ou homologue codant au moins un peptide antigénique, pour son utilisation pour vacciner du gibier d'eau.

2. rMDV pour son utilisation selon la revendication 1, ledit virus de la Maladie de Marek recombinant étant un virus de sérotype 1, de sérotype 2, ou de sérotype 3, de préférence un virus de l'Herpès de Dinde de sérotype 3 recombinant (rHVT).

3. rMDV pour son utilisation selon l'une quelconque des revendications précédentes, dans lequel la séquence nucléotidique code un peptide antigénique d'un virus de la grippe aviaire, d'un paramyxovirus aviaire de type 1 (virus de la maladie de Newcastle, NDV), d'un métapneumovirus aviaire, d'un virus de la maladie de Marek, d'un virus de la maladie de Gumboro (virus de la bursite infectieuse: IBDV), de parvovirus de l'oie et du canard de barbarie, d'un herpèsvirus de l'entérite virale du canard, d'un herpèsvirus de l'oie, d'un virus de l'hépatite de type 1, 2, et 3 de canard, d'un polyomavirus hémorragique de l'oie, d'adénovirus de canard et d'oie, de circovirus de canard et d'oie, d'un virus West Nile, de réovirus d'oie et de canard de barbarie, d'Escherichia coli, d'espèces de Salmonelle, de Pasteurella multocida, Riemerella anatipestifer, Ornithobacterium rhinotracheale, Mycoplasma gallisepticum, Mycoplasma synoviae, de microorganismes Mycoplasmas capables d'infecter des espèces de gibier d'eau, ou de coccidien.

4. Un rMDV, de préférence un rHVT, comprenant une séquence d'acide nucléique hétérologue qui code pour l'hémagglutinine d'influenza (HA), pour son utilisation comme médicament ou vaccin vétérinaire pour protéger du gibier d'eau contre la grippe.

5. rMDV pour son utilisation selon l'une quelconque des revendications précédentes, dans lequel le rMDV comprend en outre une ou plusieurs séquences nucléotidiques homologues ou hétérologues additionnelles.

6. rMDV pour son utilisation selon l'une quelconque des revendications précédentes, dans lequel le rMDV comprend en outre une autre séquence nucléotidique codant un immunomodulateur.

7. rMDV pour son utilisation selon la revendication 6, dans lequel l'immunomodulateur est une lymphokine, un interféron, ou une cytokine aviaire.

8. rMDV pour son utilisation selon l'une quelconque des revendications précédentes, dans lequel la séquence nucléotidique est insérée dans une région non-codante ou dans une région inter-ORF du génome viral.

9. rMDV pour son utilisation selon l'une quelconque des revendications précédentes, dans lequel la séquence nucléotidique est insérée dans une région non-traduite du génome viral.

10. rMDV pour son utilisation selon l'une quelconque des revendications précédentes, dans lequel la séquence nucléotidique est insérée dans une région non-traduite localisée entre UL44 et UL45, entre UL45 et UL46, entre UL41 et UL42, entre UL40 et UL41, une région localisée en aval du gène gB, entre UL53 et UL54, ou entre UL36 et UL37.

11. rMDV pour son utilisation selon l'une quelconque des revendications précédentes, dans lequel la séquence nucléotidique est sous contrôle d'un promoteur endogène ou hétérologue.

12. rMDV pour son utilisation selon l'une quelconque des revendications précédentes, dans lequel le peptide antigénique est exprimé dans des cellules de la bourse, de la rate, du thymus et des follicules des plumes des espèces de gibier d'eau vaccinées.

13. rMDV pour son utilisation selon l'une quelconque des revendications précédentes, dans lequel lesdites espèces de gibier d'eau sont vaccinées à au moins un jour d'âge.

14. rMDV pour son utilisation selon l'une quelconque des revendications précédentes, qui est administré par injection intradermique, injection sous-cutanée, injection intramusculaire, injection *in ovo,* administration orale, administration mucosale, ou par administration oculo-nasale.

15. rMDV pour son utilisation selon l'une quelconque des revendications précédentes, qui est un vaccin multivalent comprenant un mélange d'au moins deux vaccins, dont l'un desquels est le MDV recombinant tel que défini dans l'une quelconque des revendications précédentes.

16. rMDV pour son utilisation selon l'une quelconque des revendications précédentes, qui est un vaccin polyvalent comprenant deux ou plus MDV recombinants exprimant un même ou différents antigènes protéiques immunisants.

17. Vaccin pour son utilisation pour vacciner du gibier d'eau, comprenant une quantité immunisante efficace d'un virus de la Maladie de Marek recombinant (rMDV) comprenant, inséré dans le génome viral, au moins une séquence nucléotidique hétérologue ou homologue codant et exprimant dans des cellules de gibier d'eau au moins un peptide antigénique.


### SEQUENCE MISTING

<110> CEVA SANTE ANIMAL SA
<120> RECOMBINANT AVIAN HERPES VIRUS VECTORS AND VACCINE FOR IMMUNIZING WATERFOWL SPECIES
<130> 820-PCT
<150> US61/169,459
   <151> 2009-04-15
<150> US61/218,280
   <151> 2009-06-18
<150> 61/226,970
   <151> 2009-07-20
<160> 24
<170> PatentIn version 3.3
<210> 1
   <211> 8093
   <212> DNA
   <213> plasmid
<400> 1
   aagctttcaa gtgatactgc gtgatagatt taatcgccca ttggtagaaa aacatattta     60
   catgcgtgtg tgccaacgac ccgcatccgt ggatgtattg gcccctccag ttctcagcgg    120
   agaaaactac aaagcatctt gcatcgttag acatttttat cccccgggat ctgtctacgt    180
   atcttggaga cgtaacggaa acattgccac accccgcaag gaccgtgacg ggagtttttg    240
   gtggttcgaa tctggccgcg gggccacact agtatccaca ataaccctcg gaaactctgg    300
   actcgaatct cctccaaagg tttcctgctt ggtagcgtgg aggcaaggcg atatgataag    360
   cacatcgaat gctacagctg taccgacggt atattatcac ccccgtatct ctctggcatt    420
   taaagatggg tatgcaatat gtactataga atgtgttccc tctgggatta ctgtgaggtg    480
   gttagttcat gatgaacccc agcctaacac aacttatgat actgtggtta caggtctctg    540
   caggaccatc gatcgttata gaaatctcgc cagtcggatt ccagtccagg acaactgggc    600
   gaaaacgaag tatacgtgca gactaattgg atatccgttc gacgtggata gatttcaaaa    660
   ttccgaatat tatgatgcaa cgccgtcggc aagaggaatg ccgatgattg taacaattac    720
   ggccgttcta ggactggcct tgtttttagg tattggtatc attatcacag ccctatgctt    780
   ttacctaccg gggcggaatt aagattaacc atcgtatgtg atataaaaat tattaagtgt    840
   tataaccgat cgcattcttc tgtttcgatt cacaataaat aaaatggtat tgtaatcagc    900
   accatcgcat tgtttcgtag atgactcatg ttcagtccgc gtgatgtcaa aaatacgtat    960
   ttttggtatc acgcagcggc caaaatgccc attatgttat ttttactcca aacgcggtat   1020
   ttaaaacatc gggacgtaca tcatgtggcg cacgttaatc gtatacggtg ccgctacatt   1080
   aaaaatcgca agtctccgaa tatcaagctc acggccaaaa cgtcggtaat aatcttacgc   1140
   atcgaatgtg atacggatac cgtacaatcg ctgagtagat ttcctatata gttactcagt 1200
   agtgatacac aatcacaaaa tcgctggggt atatcatata agaatgatgt cgcccacccc 1260
   tgaagatgat cgcgatctcg ttgtggttcg tggacgtctc cgaatgatgg atagcggcac 1320
   ggaaacagat agagagcaac gacatccacg tacgacttgg cgatcgatct gttgtgggtg 1380
   tacgatagga atggtattta ccatattcgt tctcgtagcg gcagtattgt tgggatcact 1440
   attcactgtt tcatacatgg ccatggaatc gggaacatgt cccgatgaat ggattggttt 1500
   gggttatagt tgcatgcgcg tggccgggaa aaatgcaact gatcttgagg cgttggatac 1560
   atgtgctcgg cataacagca aacttattga cttcgcaaac gccaaagttc tggttgaagc 1620
   tatcgcccca ttcggtgtgc caaatgcagc atatggggaa gtcttccggt taagggacag 1680
   caaaaccacg tgtatacgac ctaccatggg aggacccgtg tcggcagact gtcctgtaac 1740
   atgtaccgtt atatgtcagc gacccaggcc tctaagtacc atgtcttcca tcattagaga 1800
   tgcccgcgtg tatcttcatt tagaacgacg cgattattat gaagtctacg cctctgtcct 1860
   ctctaatgcg atgagtaaat aggccttatt ggcctaagat acattgatga gtttggacaa 1920
   accacaacta gaatgcagtg aaaaaaatgc tttatttgtg aaatttgtga tgctattgct 1980
   ttatttgtaa ccattataag ctgcaataaa caagttaaca acaacaattg cattcatttt 2040
   atgtttcagg ttcaggggga ggtgtgggag gttttttaaa gcaagtaaaa cctctacaaa 2100
   tgtggtatgg ctgattatga tcatgaacag actgtgagga ctgaggggcc tgaaatgagc 2160
   cttgggactg tgaatcgtcg acttaaatgc aaattctgca ttgtaacgat ccattggagc 2220
   acatccataa agatagacca gccaccatga ttgccagtgc tagggagctc gccactgttg 2280
   aataaattga cagtatttgg taggttccta ttgattccaa ttttactcca cttatttcct 2340
   ctctttttaa tcttgcttct tctgaatact gcgggtagtc atacgttccg tttcttacac 2400
   tttccataca ttcattatca catctgtgat agaactcgaa acaaccgtta ccaagctcct 2460
   ttgcattatc cctaagctgt agtcggacct tgtcgtaaag gttcttgaca tttgagtcat 2520
   gaaagtctag agttctctca ttttccatga gaaccagaag ttcagcatta taagtccaga 2580
   catctaggaa tccgtcttcc atcttcttgt ttaaattttc tattctcctt tctaatttat 2640
   taaattcctt tccaacggcc tcaaactgag tgttcatttt gtcaatgatc gagttgacct 2700
   tattggtgac tccatctatt gccttttgag tggattcttt gtctgcagcg tacccactcc 2760
   cctgctcgtt gctatggtgg tacccatacc aaccatctac cattccctgc catcctccct 2820
   ctataaaacc tgctatagct ccaaatagtc ctcttgtctc tctttgaggg ctatttctga 2880
   gcccagtcgc aaggactaat ctgtttgatt tcacatattt ggggcattcc ccgatggtga 2940
   gagggtggat gttgtggaat ggcatactag agtttatcgc ccctattgga gtttgacact 3000
   tggtgttgca gttaccatat tccaatttac ttttcataat tgttgagtcc cctttcttga 3060
   caattttgta tgcattttct ggagcaatga aatttccatt actctcaaag tttattgcat 3120
   cattcggttt taaaattgtc caaaagaact ccatccttcc actttgcccg tttaccttgg 3180
   atctagtagc tatttttggt accaatctct ggtttagtgt tgatgtccca acggaaatat 3240
   aggtggttgg gttttgatag agccttgtct gctctgccgc atcatttgga tggtgaatcc 3300
   cccacagtac caaaagatct tcttggttgg tattattgta acttctcttt attgttgggt 3360
   atgcattgtc ctttttgata agccatacca catttctaaa aaaggaggac cttccctggt 3420
   atggacatgc tgagctcacc cctgatgagg cttcatgatc tgaccaagaa cttttgggga 3480
   tgatctgaat tttctcaaaa tggtttattc tgctcaatag gtgtttcagt tcttcatagt 3540
   cgttgaaatt ccctgggtaa cagaggtcat tggctggatt gatcttctcc actatgtaag 3600
   accattccgg cacattgagg aattcgtcac acattgggtt cccgaggagc catccagcta 3660
   cactacaatc tcttaaaatt agaggcttta ctccatctag atcgcagagc ttcccgttgt 3720
   gtgtcttttc cagtatgtct tgggcgtgtg taacagtgac gttcttttcc attattgtgt 3780
   caacctgctc tgtcgagttg tttgcatggt aaccaatgca aatctgatca cttttaacaa 3840
   gactgactat tgcaaaaaga agcactattt tctccatgga tcctctagag gatctgacgg 3900
   ttcactaaac cagctctgct tatatagacc tcccaccgta cacgcctacc gcccatttgc 3960
   gtcaatgggg cggagttgtt acgacatttt ggaaagtccc gttgattttg gtgccaaaac 4020
   aaactcccat tgacgtcaat ggggtggaga cttggaaatc cccgtgagtc aaaccgctat 4080
   ccacgcccat tgatgtactg ccaaaaccgc atcaccatgg taatagcgat gactaatacg 4140
   tagatgtact gccaagtagg aaagtcccat aaggtcatgt actgggcata atgccaggcg 4200
   cgccatttac cgtcattgac gtcaataggg ggcgtacttg gcatatgata cacttgatgt 4260
   actgccaatg ggcagtttac cgtaaatact ccacccattg acgtcaatgg aaagtcccta 4320
   ttggcgttac tatgggaaca tacgtcatta ttgacgtcaa tgggcggggg tcgttgggcg 4380
   gtcagccggc gggccattta ccgtaagtta tgtaacgcgg aactccatat atgggctatg 4440
   aactaatgac cccgtaattg attactatta ataactctgc agccttattg gccaaaacac 4500
   acctctaacg gttactgtgt tttatttatc caatcacacc atagacatta taaacataat 4560
   atggatcttt atttcatata atgtattata cagaccgcgc atcgcgtgta aaatattatt 4620
   cgctggacac tttcagccta cgtaaaatat gttctacctt ggacactggt atatctccac 4680
   gcattgtttt ttcatgaaag tatttggttg tcggagtaga caccctgcca gattccgaaa 4740
   ctggagggtc attgctcgcc aaagaaggcc ccccagaccc atcggtctta cgaattgcac 4800
   cagtcagacc gtttccgatg ttcgcacatt gagaactcga ccccgccccc gcaaattcag 4860
   tcaaaccagg aaccgtatcg ccacagccac attcgacacg atcgcctaat tgatgtgtgg 4920
   gcgtacttcc cgacttgttc gagtttttat cttgcttccg aatagatttc ataagccgtg 4980
   cattaacatc gcttattcgc tttggcagta attttatccc cagtagagcc agggcgttct 5040
   tagacagttt aacatcatcg gttgcagcgc ggccgaacca agtatttatg ctgcactcga 5100
   gagcccccca tgacacgggc aacatgctcg gaaacaaatt cccaagctga tgttcaaatt 5160
   ggtcttgctg caacagggcc tcttttaata tagtattttc cgtattaccg tccaaccata 5220
   tcacatatcc cctctgagtt aaattaatta ggtactgaca atgatggttg agaagtgaaa 5280
   ccaacgaaac cactgctagt atggcacgtg agttccgatc accatcaaag tccaccaggc 5340
   gggtttgcga ccataatacg ctggtttgta gtaagggggc gatgacattg cctataattc 5400
   gacaaagcaa tgatgtcgaa ctccatttgt ctgatggtaa ggaggaaatt tcttccatga 5460
   attcgtaatc atggtcatag ctgtttcctg tgtgaaattg ttatccgctc acaattccac 5520
   acaacatacg agccggaagc ataaagtgta aagcctgggg tgcctaatga gtgagctaac 5580
   tcacattaat tgcgttgcgc tcactgcccg ctttccagtc gggaaacctg tcgtgccagc 5640
   tgcattaatg aatcggccaa cgcgcgggga gaggcggttt gcgtattggg cgctcttccg 5700
   cttcctcgct cactgactcg ctgcgctcgg tcgttcggct gcggcgagcg gtatcagctc 5760
   actcaaaggc ggtaatacgg ttatccacag aatcagggga taacgcagga aagaacatgt 5820
   gagcaaaagg ccagcaaaag gccaggaacc gtaaaaaggc cgcgttgctg gcgtttttcc 5880
   ataggctccg cccccctgac gagcatcaca aaaatcgacg ctcaagtcag aggtggcgaa 5940
   acccgacagg actataaaga taccaggcgt ttccccctgg aagctccctc gtgcgctctc 6000
   ctgttccgac cctgccgctt accggatacc tgtccgcctt tctcccttcg ggaagcgtgg 6060
   cgctttctca tagctcacgc tgtaggtatc tcagttcggt gtaggtcgtt cgctccaagc 6120
   tgggctgtgt gcacgaaccc cccgttcagc ccgaccgctg cgccttatcc ggtaactatc 6180
   gtcttgagtc caacccggta agacacgact tatcgccact ggcagcagcc actggtaaca 6240
   ggattagcag agcgaggtat gtaggcggtg ctacagagtt cttgaagtgg tggcctaact 6300
   acggctacac tagaaggaca gtatttggta tctgcgctct gctgaagcca gttaccttcg 6360
   gaaaaagagt tggtagctct tgatccggca aacaaaccac cgctggtagc ggtggttttt 6420
   ttgtttgcaa gcagcagatt acgcgcagaa aaaaaggatc tcaagaagat cctttgatct 6480
   tttctacggg gtctgacgct cagtggaacg aaaactcacg ttaagggatt ttggtcatga 6540
   gattatcaaa aaggatcttc acctagatcc ttttaaatta aaaatgaagt tttaaatcaa 6600
   tctaaagtat atatgagtaa acttggtctg acagttacca atgcttaatc agtgaggcac 6660
   ctatctcagc gatctgtcta tttcgttcat ccatagttgc ctgactcccc gtcgtgtaga 6720
   taactacgat acgggagggc ttaccatctg gccccagtgc tgcaatgata ccgcgagacc 6780
   cacgctcacc ggctccagat ttatcagcaa taaaccagcc agccggaagg gccgagcgca 6840
   gaagtggtcc tgcaacttta tccgcctcca tccagtctat taattgttgc cgggaagcta 6900
   gagtaagtag ttcgccagtt aatagtttgc gcaacgttgt tgccattgct acaggcatcg 6960
   tggtgtcacg ctcgtcgttt ggtatggctt cattcagctc cggttcccaa cgatcaaggc 7020
   gagttacatg atcccccatg ttgtgcaaaa aagcggttag ctccttcggt cctccgatcg 7080
   ttgtcagaag taagttggcc gcagtgttat cactcatggt tatggcagca ctgcataatt 7140
   ctcttactgt catgccatcc gtaagatgct tttctgtgac tggtgagtac tcaaccaagt 7200
   cattctgaga atagtgtatg cggcgaccga gttgctcttg cccggcgtca atacgggata 7260
   ataccgcgcc acatagcaga actttaaaag tgctcatcat tggaaaacgt tcttcggggc 7320
   gaaaactctc aaggatctta ccgctgttga gatccagttc gatgtaaccc actcgtgcac 7380
   ccaactgatc ttcagcatct tttactttca ccagcgtttc tgggtgagca aaaacaggaa 7440
   ggcaaaatgc cgcaaaaaag ggaataaggg cgacacggaa atgttgaata ctcatactct 7500
   tcctttttca atattattga agcatttatc agggttattg tctcatgagc ggatacatat 7560
   ttgaatgtat ttagaaaaat aaacaaatag gggttccgcg cacatttccc cgaaaagtgc 7620
   cacctgacgt ctaagaaacc attattatca tgacattaac ctataaaaat aggcgtatca 7680
   cgaggccctt tcgtctcgcg cgtttcggtg atgacggtga aaacctctga cacatgcagc 7740
   tcccggagac ggtcacagct tgtctgtaag cggatgccgg gagcagacaa gcccgtcagg 7800
   gcgcgtcagc gggtgttggc gggtgtcggg gctggcttaa ctatgcggca tcagagcaga 7860
   ttgtactgag agtgcaccat atgcggtgtg aaataccgca cagatgcgta aggagaaaat 7920
   accgcatcag gcgccattcg ccattcaggc tgcgcaactg ttgggaaggg cgatcggtgc 7980
   gggcctcttc gctattacgc cagctggcga aagggggatg tgctgcaagg cgattaagtt 8040
   gggtaacgcc agggttttcc cagtcacgac gttgtaaaac gacggccagt gcc 8093
<210> 2
   <211> 25
   <212> DNA
   <213> primer
<400> 2
   ccccgaattc atggaagaaa tttcc 25
<210> 3
   <211> 40
   <212> DNA
   <213> Primer
<400> 3
   cgcgcgcctt attggccaaa acacacctct aacggttact 40
<210> 4
   <211> 39
   <212> DNA
   <213> Primer
<400> 4
   gcgcggccaa taaggccaaa cacagtaacc gttagaggt 39
<210> 5
   <211> 28
   <212> DNA
   <213> Primer
<400> 5
   ccccaagctt tcaagtgata ctgcgtga 28
<210> 6
   <211> 37
   <212> DNA
   <213> Primer
<400> 6
   gcgcggccaa taaggccaac atcgggacgt acatcat 37
<210> 7
   <211> 40
   <212> DNA
   <213> Primer
<400> 7
   gcgcggcctt attggcctta aataccgcgt ttggagtaaa 40
<210> 8
   <211> 25
   <212> DNA
   <213> Synthetic DNA
<400> 8
   agctgccccc cccggcaagc ttgca 25
<210> 9
   <211> 17
   <212> DNA
   <213> Synthetic DNA
<400> 9
   tcgacatttt tatgtac 17
<210> 10
   <211> 21
   <212> DNA
   <213> Synthetic DNA
<400> 10
   aattcggccg ggggggcagc t 21
<210> 11
   <211> 13
   <212> DNA
   <213> Synthetic DNA
<400> 11
   gcccccccgg ccg 13
<210> 12
   <211> 20
   <212> DNA
   <213> Synthetic DNA
<400> 12
   agcttgccaa taaggctgca 20
<210> 13
   <211> 20
   <212> DNA
   <213> Synthetic DNA
<400> 13
   atggcccgcc ggctgaccgc 20
<210> 14
   <211> 20
   <212> DNA
   <213> Synthetic DNA
<400> 14
   gcggtcagcc ggcgggccat 20
<210> 15
   <211> 22
   <212> DNA
   <213> Synthetic DNA
<400> 15
   ggtaaactgc agacttggca gt 22
<210> 16
   <211> 22
   <212> DNA
   <213> Synthetic DNA
<400> 16
   actgccaagt ctgcagttta cc 22
<210> 17
   <211> 22
   <212> DNA
   <213> Synthetic DNA
<400> 17
   ggcataatgc atggcgggcc at 22
<210> 18
   <211> 22
   <212> DNA
   <213> Synthetic DNA
<400> 18
   atggcccgcc atgcattatg cc 22
<210> 19
   <211> 21
   <212> DNA
   <213> Primer
<400> 19
   cgggagctct aattgtttgt g 21
<210> 20
   <211> 20
   <212> DNA
   <213> Primer
<400> 20
   cgggaattcg cttacaattt 20
<210> 21
   <211> 21
   <212> DNA
   <213> Primer
<400> 21
   ccggggccct aattgtttgt g 21
<210> 22
   <211> 20
   <212> DNA
   <213> Primer
<400> 22
   cggggtaccg cttacaattt 20
<210> 23
   <211> 24
   <212> DNA
   <213> Primer
<400> 23
   gcaagcttgc gatgacgaac ctgc 24
<210> 24
   <211> 25
   <212> DNA
   <213> Primer
<400> 24
   gcgtcgactc acctccttag ggccc 25